# EUROPEAN PATENT APPLICATION

(11) **EP 4 838 403 A1**
(43) Date of publication of application: **11.11.2026**
(21) Application number: 26173908.0
(22) Date of filing: 22.04.2026
(51) Int. Cl.: G06F 40/284, A61B 5/00, A61B 5/28, A61B 5/341, A61B 5/349, A61B 5/358, G16H 50/20, G16H 50/30, G16H 50/70

(54) **SYSTEMS, DEVICES, COMPONENTS AND METHODS FOR QUANTUM WAVE FUNCTION TOKENIZERS IN MEDICAL DEVICES**

(30) Priority: 07.05.2025 US 202563801711 P; 23.07.2025 US 202563849757 P; 02.08.2025 US 202563856390 P; 03.08.2025 US 202563856650 P; 18.03.2026 US 202619570305
(71) Applicant: WaveMedix, Inc., 1807 Blonay (CH)
(72) Inventor: RUPPERSBERG, Peter, 1807 Blonay (CH); STINGELE, Francesca, 1807 Blonay (CH)
(74) Representative: Ipsilon Luxembourg

(57) **Abstract**

A plurality of electrocardiogram (ECG) signals received from a patient are vectorized into vectorized data sets. Parametric wave functions are fitted to each of the vectorized data sets, thereby generating corresponding fitted wave function data sets. Each fitted wave function data set includes an amplitude parameter, a phase parameter, an angle parameter, a width parameter, or a location parameter of a corresponding ECG signal. A set of quantum wave tokens corresponding to each of the fitted wave function data sets are generated. Each quantum wave token comprises at least one of: an amplitude value, a phase value, an angle value, a width value, and a location value. At least some of the quantum wave tokens are provided to one or more machine learning models for at least one of: filtering, processing, transforming, analyzing, or diagnosing at least some of the ECG signals.

## Description

### PRIORITY DATA

This application claims benefit and is a utility U.S. Patent Application of U.S. Provisional Application No. 63/801,711, filed on May 7, 2025, entitled "Systems, Devices, Components and Methods for Quantum Wave Function Tokenizers in Medical Devices", U.S. Provisional Application No. 63/849,757, filed on July 23, 2025, entitled "Systems, Devices, Components and Methods for Quantum Wave Function Tokenizers in Medical Devices", U.S. Provisional Application No. 63/856,390, filed on August 2, 2025, entitled "Systems, Devices, Components and Methods for Quantum Wave Function Tokenizers in Medical Devices and for Providing Medically Safe Cardiac Diagnoses for Patients", and U.S. Provisional Application No. 63/856,650, filed on August 3, 2025, entitled "Systems, Devices, Components and Methods for Quantum Wave Function Tokenizers in Medical Devices and for Providing Medically Safe Cardiac Diagnoses for Patients", the disclosures of each of which are incorporated herein by reference in their respective entireties.

### BACKGROUND

In the field of modern medical devices, various tools have been developed to help provide patients diagnostics. As an example, an electrocardiogram ("ECG") is a non-invasive test that records the electrical activity of the heart over a period of time using electrodes placed on the skin of a patient. It provides a visual representation (usually as a trace on paper or on a screen) of how the patient's heart is beating and conducting electrical impulses. ECGs are commonly used in medicine because they can quickly provide valuable insights into a patient's cardiac health and condition, are often inexpensive to acquire, widely available, and can be critical in triaging and managing cardiac emergencies. For example, ECGs are widely used to diagnose, monitor and provide prognoses regarding the state or condition of a patient's heart. Some applications of ECGs include diagnosing cardiac conditions such as arrhythmias (e.g., atrial fibrillation, ventricular tachycardia, heart blocks, etc.), whether a myocardial infarction or heart attack has occurred in a patient by identifying ischemia or infarction through ST-segment changes, whether a patient suffers from pericarditis or myocarditis by identifying inflammation-related ECG changes, or whether a patient suffers from electrolyte imbalances such as hyperkalemia or hypokalemia (which can produce characteristic changes in ECG signals), and whether a patient might have structural abnormalities such as left ventricular hypertrophy or atrial enlargement.

However, reliable and accurate interpretation of ECGs is tricky and complicated and may require experts with lengthy and extensive training and education. Even when ECGs are interpreted by experts and in context (e.g., alongside patient clinical history, physical examination, and often imaging or lab tests), errors in ECG interpretation still remain relatively common. What is needed are improved, and much more reliable and accurate, means and methods of acquiring, processing, and diagnosing ECG data on the spot (e.g., in a cardiologist's office) over a short periods of time, and with much greater precision than is currently possible.

### BRIEF DESCRIPTION OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In the figures, elements having the same designation have the same or similar functions.
Fig. 1 illustrates a framework of a dual LLM architecture according to various aspects of the present disclosure.
Figs. 2-4 illustrate various training/learning processes involving the dual LLM architecture according to various aspects of the present disclosure.
Fig. 5 illustrates a process flow for generating quantum wave tokens according to various aspects of the present disclosure.
Fig. 6 illustrates an example artificial neural network according to various aspects of the present disclosure.
Figs. 7-8 illustrate flowcharts of methods according to various aspects of the present disclosure.
Fig. 9 illustrates a computer system according to various aspects of the present disclosure.

Embodiments of the present disclosure and their advantages are best understood by referring to the detailed description that follows. It should be appreciated that like reference numerals are used to identify like elements illustrated in one or more of the figures, wherein showings therein are for purposes of illustrating embodiments of the present disclosure and not for purposes of limiting the same.

### DETAILED DESCRIPTION

It is to be understood that the following disclosure provides many different embodiments, or examples, for implementing different features of the invention. Specific examples of components and arrangements are described below to simplify the present disclosure. These are, of course, merely examples and are not intended to be limiting. Various features may be arbitrarily drawn in different scales for simplicity and clarity.

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

In the field of cardiac monitoring, ECGs are used in emergency departments, operating rooms, and intensive care units to continuously monitor heart function. ECGs are also often acquired using ambulatory ECG equipment (e.g., Holter monitors) to record ECGs over 24-48 hour time periods to detect intermittent arrhythmias or other heart problems. More typically and much more often, however, 12-lead ECGs are acquired from a patient using a conventional ECG machine located in a physician's or cardiologist's office. There, ECGs are usually recorded over a time period of 10-15 seconds or so. It is based upon this very short time period over which ECG signals are sensed and then recorded an/or presented on a strip of paper or other display that the physician's or cardiologist diagnoses the patient's current state of cardiac health. If a cardiac signal anomaly or problem (e.g., transient arrhythmias or ischemia) should appear outside this very short diagnostic window, it will not be seen or diagnosed by the physician or cardiologist. For various reasons, such as physician time and availability, and computational time and other limits, longer ECG signal recordings are rarely performed in a cardiologist's office. As a result, it is not unusual for important aspects of a patient's cardiac status and health to be missed altogether during an office visit to the physician or cardiologist.

The use of ECGs in diagnostics may also have various other limitations. For example, ECGs are frequently unable to reveal the early stages of cardiac disease. ECGs are also not useful for diagnosing all heart conditions (e.g., structural issues such as heart valve problems, which may require echocardiography or MRI for proper diagnosis). ECGs can also have limited sensitivity and specificity. For example, a patient with a "normal ECG" may still have a serious cardiac condition (e.g., angina or structural heart disease). False positives can occur in ECGs, where changes in ECG signals can mimic myocardial infarction or ischemia but be caused by other things such as early repolarization, pericarditis, or patient anxiety. Accurate or reliable interpretation of ECGs also presents challenges, since ECG interpretation requires considerable expertise, training and education, and misinterpretation of ECGs can lead to incorrect diagnosis or missed conditions.

Moreover, ECG signals obtained from patients can vary significantly with patient age, body physique, ECG lead placement, ECG lead coupling, patient electrolyte levels, and medications a patient might be taking. Technical Limitations such as poor electrode placement or patient movement can introduce unwanted artifacts in ECG signals. ECG signal baseline wander, electrical interference, or muscle tremors can also obscure key waveforms. ECGs show electrical activity of the heart, not direct structural or mechanical function of the heart, and therefore are typically unable to be used to assess heart valve function, wall motion abnormalities, or blood flow (which instead typically require imaging techniques such as echocardiography or MRI). Changes observed in a patient's ECG signals can also be non-specific and attributable to multiple conditions. For example, ST-segment depression can be due to any one or more of ischemia, electrolyte imbalance, or digitalis effect. Variability and ambiguity can also be present in ECG signals, where normal variants (e.g., early repolarization) can mimic pathology. Bundle branch blocks or pre-excitation can also obscure signs of ischemia or infarction. In obese or muscular individuals, ECG signals can be more difficult to interpret. Pediatric ECGs sometimes follow different patterns, and may require specialist knowledge and expertise.

In order to address these issues discussed above, the present disclosure introduces a quantum wave function tokenizer for processing medical data (e.g., including but not limited to ECG data) and a dual Large Language Model (LLM) architecture to generate fast and accurate diagnoses based on the medical data. In more detail, LLMs are transformer-based artificial intelligence (AI) models that when fine-tuned, can make meaningful predictions and decisions based on smaller but focused datasets. The LLMs of the present disclosure are deployed in a lightweight and interconnected system designed specifically for medical applications. By combining the strengths of transformer models with scalable, real-time processing, the present disclosure bridges the gap between what is possible and what is practical in medical AI. This allows for smarter diagnostics, more personalized treatments, and ultimately better outcomes for patients, even in specialized or underexplored areas of medicine.

In some embodiments, the system is structured around two AI models that collectively form an advanced cardiovascular diagnostic and risk prediction platform. The first model operates as a real-time ECG feature extractor (using, for example, a quantum wave tokenizer), while the second model functions as a clinical reasoning system, capable of analyzing long-duration ECGs, integrating patient history, and generating clinically validated risk assessments. The second model may also use a Retrieval Augmentation Generation module (or RAG). Retrieval-Augmented Generation (RAG) is a process that enhances the capabilities of LLMs by allowing them to access and incorporate external knowledge bases before generating responses. Instead of relying solely on its own training data, RAG enables LLMs to retrieve relevant information from a separate knowledge store, making their outputs more accurate, context-aware, and up-to-date. In some embodiments, the RAG comprises a machine learning model that may be configured to combine two key components: (a) a retrieval module configured to search a large external or internal knowledge base (e.g., a document or literature database, a private dataset, etc.) to find relevant pieces of information related to an input query, and (b) a generation module configured to use a language model (e.g., GPT) to generate a response based on both the input query and the retrieved documents.

One advantage of such a system is its second model, which is not merely an AI-driven inference engine, but may also be explicitly trained using real-world clinical trial data, ensuring that its predictive capabilities are grounded in actual patient outcomes. In one embodiment, the first model is trained using a dataset of, for example, 46,000 patients, with an initial fine-tuning subset of 5,000 patients, and outputs a structured 54-dimensional diagnostic probability vector for every 10-second ECG segment. In one embodiment, these 54 categories can include:
- 49 predefined clinical diagnoses, extracted from labeled ECG data;
- 5 additional diagnostic categories, introduced through analytic preprocessing, which quantify T-wave and P-wave variability, amplitude fluctuations, and other waveform-derived parameters.

Unlike conventional ECG models that produce hard classifications, in one embodiment such a system leverages a softmax-based probability distribution across all 54 diagnoses (this specific number is merely a non-limiting example), ensuring that multiple potential conditions can be recognized simultaneously. This feature transforms the diagnosis space into a structured feature representation, functioning as a soft embedding that retains clinically relevant information while remaining fully interpretable.

Softmax is a mathematical function often used in machine learning, especially in classification tasks. It takes a vector of raw scores (also called logits) and transforms them into probabilities that sum to 1. This makes it useful for predicting the probability distribution over multiple classes. Each output value is between 0 and 1. The outputs add up to 1, forming a valid probability distribution. Softmax emphasizes the largest scores, making them stand out more (useful for classification). For example, if a neural network predicts raw scores like [2.0, 1.0, 0.1] for 3 classes, softmax converts these into probabilities like [0.65, 0.24, 0.11].

The second model extends this approach by:
- Processing long-duration ECG recordings (for example up to 30 minutes) by aggregating multiple 10-second feature vectors into a cohesive, large-scale diagnostic and risk evaluation.
- Integrating patient-specific risk factors, including age, body mass index (BMI), history of hypertension, diabetes, and other comorbidities.
- Retrieving and comparing historical ECG reports, enabling direct trend analysis over multiple visits
- Generating structured clinical summaries, mirroring physician-style diagnostic reports that can be stored and retrieved for long-term patient tracking.

However, in some embodiments, an important function of the second model is its ability to predict future cardiovascular risks, including, but not limited to:
- Risk of atrial fibrillation within 5 years
- Probability of ventricular tachycardia recurrence
- Risk of sudden cardiac death
- Likelihood of heart attack recurrence or progression to heart failure
Unlike traditional ECG analysis, which is focused on current-state diagnostics, such a system is designed for predictive medicine. The ability to provide quantified risk scores for future cardiovascular events makes it a fundamentally new class of AI-driven medical technology, bridging the gap between pattern recognition and true clinical decision-making.

In some embodiments, the second model's predictive capabilities (which in addition to LLM architecture may also include convolutional neural network features, circuitry, programming, training and learning), are not trained on theoretical assumptions but are directly fine-tuned using real-world clinical trial data. According to one embodiment, the training process follows a three-phase clinical validation strategy:
1. Retrospective Study Using Large-Scale Historical Patient Data
   ∘ The initial phase involves fine-tuning the second model on retrospective clinical data, where ECGs, patient histories, and long-term outcomes are already known.
   ∘ By mapping past ECG feature spaces to actual cardiac event outcomes, the model learns how specific ECG abnormalities and patient parameters correlate with future disease progression.
   ∘ This phase allows the second model to develop baseline risk stratification capabilities before any prospective data is collected.
2. Prospective Clinical Trial with Real-Time Patient Monitoring
   ∘ The second phase includes an active clinical trial, where patients are monitored over time, and their ECG-derived risk predictions are compared with actual cardiac events that occur in the future.
   ∘ This is where the model achieves its greatest refinement, as it continuously learns how well its predictions align with real-world patient outcomes.
   ∘ If an ECG pattern suggests a 70% probability of atrial fibrillation onset within 5 years, but the actual occurrence rate in the monitored population is higher or lower, the model undergoes dynamic recalibration to ensure its outputs are statistically optimized for real-world applications.
3. Post-deployment Validation and Expansion to New Risk Models
   ∘ Once the system is deployed in clinical settings, additional post-market validation studies allow further refinement.
   ∘ New patient cohorts, risk groups, and emerging cardiac conditions can be incorporated into the model, allowing continuous learning and expansion.
Unlike AI models that rely solely on pre-existing datasets, the above approach of the present disclosure ensures that the AI model herein:
- Is trained on real-world cardiac event progression data, not just static ECG interpretations.
- Continuously learns and adapts to new clinical insights over time.
- Achieves regulatory-grade validation, making it viable for adoption in hospitals, research centers, and telemedicine applications.
- According to some embodiments, the integration of ECG waveform analysis, patient history modeling, and clinical trial- based predictive training ensures that the system herein is capable of:
   1. Real-time feature extraction from 10-second ECG segments (via the first model).
   2. Large-scale ECG aggregation and patient history integration (via the second model).
   3. Clinical-grade risk prediction for major cardiac events.
   4. Structured report generation, suitable for long-term patient records.
   5. Longitudinal ECG tracking, detecting trends across multiple visits.
   6. Dynamic recalibration based on clinical trial results, ensuring long-term predictive accuracy.

The AI system of the present disclosure enables short-term context handling via transformer attention (e.g., a first LLM) for real-time reasoning, as well as long-term memory handling by storing structured, human-readable memory (e.g., ASCII text) for full transparency and control. With respect to memory retrieval, the present disclosure utilizes structured memory lookup using explicit retrieval strategies. With respect to memory updating, the present disclosure utilizes fine-tuned memory learning in a second LLM that is optimized for transparency and expert control. The memory is stored as explicit knowledge in human-readable, editable memory logs, and it is adaptable without retraining by continuously updating stored knowledge. The memory is stored in a structured text format, ensuring full transparency. The memory is scalable, as modular ASIC architecture enables high-speed, structured recall. The structured memory also ensures domain-specific correctness (e.g., medicine, law, robotics, etc). Due to the above features, the memory retrieval of the present disclosure is efficient and low-cost, as structured queries limit unnecessary retrieval, and the system is particularly well-suited for medical AI that must ensure correct, demonstrate mathematical proof, and/or have an enterprise AI knowledge base, and it is the only system that provides structured, expert-validated memory, making it ideal for high-stakes applications.

The present disclosure also uses quantum wave functions to enable the LLMs in medical data processing with precision. Specifically, raw signals, such as ECGs or medical images, are transformed into structured data (e.g., tokens) using quantum wave representations. Each token captures critical parameters of a quantum wave-e.g., amplitude, angle, time, period, and phase-allowing for a rich, multidimensional description of multidimensional structures that would otherwise be unavailable for use by an LLM or a Retrieval Augmentation Generation module (or RAG).

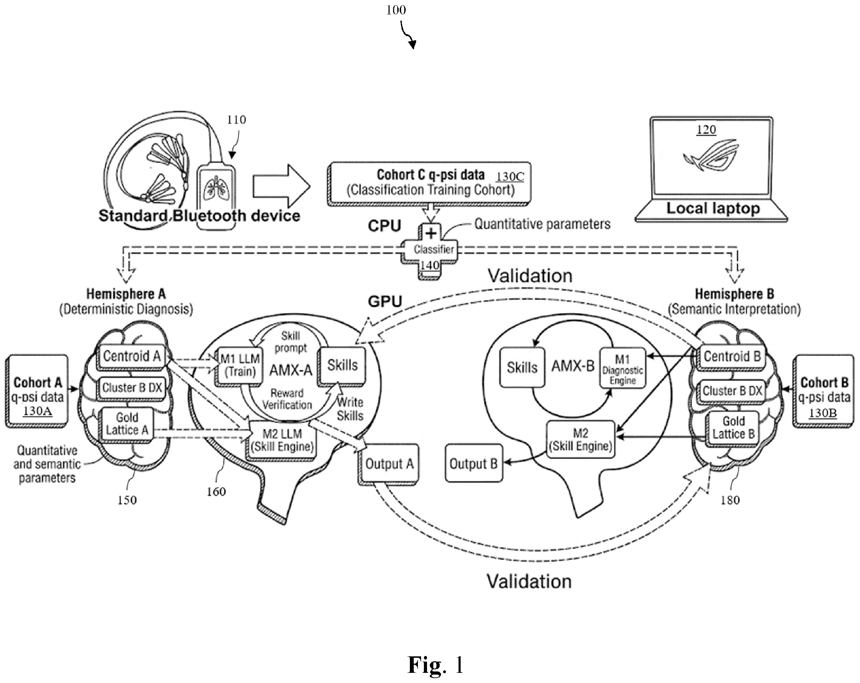

The various aspects of the present disclosure are discussed in more detail below with reference to Figs. 1-9.

Fig. 1 illustrates an example framework 100 in which the dual LLM architecture of the present disclosure is illustrated. In some embodiments, the framework 100 enables the implementation of standalone AI-driven 12-lead ECG machine that can:
- Offer real-time AI interpretation directly on the device, without requiring cloud connectivity.
- Support longer-duration ECGs (e.g., 1-5 minutes) for deeper AI analysis of transient arrhythmias.
- Provide actionable AI insights immediately, without relying on post-processing.
- Have built-in patient records and memory for tracking ECG changes over time.
- Work as a self-contained medical device, allowing it to be deployed in clinics, urgent care, or even ambulances without external dependencies.

The framework 100 includes a wireless communications device 110 that is configured to generate patient medical data. In some embodiments, the wireless communications device 110 includes a Bluetooth-enabled medical device, such as a Bluetooth-enabled ECG device with a plurality of electrodes. As non-limiting examples, the Bluetooth-enabled ECG device may include a SmartHeart^{®} Wireless 12-Lead ECG, a QT Medical PCA-500 12-Lead ECG Recorder, a single-lead or 6-lead handheld ECG from AliveCor^{™} KardiaMobile^{™}, a wearable single-channel Holter-style ECG streaming device from QardioCore^{™}, or other small 1-lead or 3-lead ECG devices from Beurer^{™} or Zive^{™}.

Regardless of the exact implementation of the wireless communications device 110, the electrodes thereof may be placed on a body of a patient, and the corresponding ECG data may then be transmitted wirelessly to one or more nearby Bluetooth-enabled devices. One of these nearby devices may include a computer 120, which is also a part of the framework 100. In some embodiments, the computer 120 includes a local laptop computer, but it is understood that the computer 120 may include a desktop computer, a tablet computer, or even a smartphone in other embodiments. The computer 120 has one or more processing units, such as Central Processing Units (CPUs) and/or Graphics Processing Units (GPUs), that are configured to train and/or run machine learning models, such as LLMs. In some embodiments, the computer 120 includes an autonomous AI agent that is configured to interact with the wireless communications device 110 directly. Note that in some embodiments, the wireless communication device 110 may be replaced by a wired communication device, such as a wired ECG machine having a plurality of electrodes, in which case the ECG data may still be transmitted through wires to the computer 120.

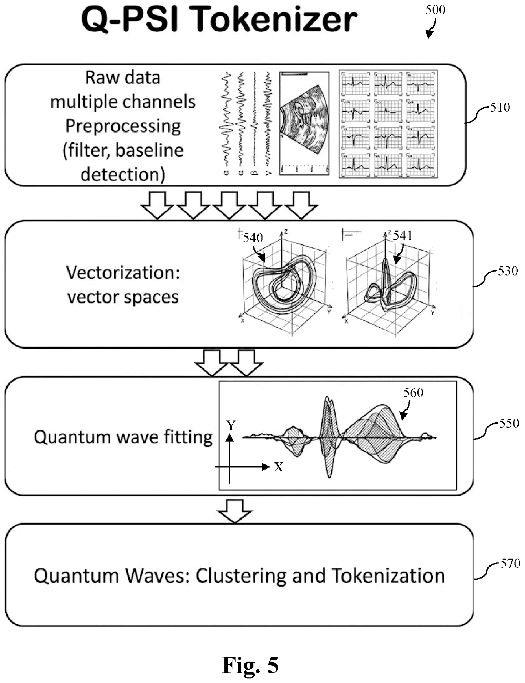

According to the framework 100, a Quantum Wave Function Tokenizer (Q-PSI) is used to convert the raw patient medical data (e.g., ECG data) into precise, processable, understandable, clinically interpretable parameters. This conversion process involves fitting the raw patient medical data with quantum waves and subsequently processing them with an interpreter, as will be discussed in more detail below with reference to Fig. 5.

In the illustrated embodiment of Fig. 1, the data generated by the Q-PSI includes cohort data obtained from thousands of patients and split into different cohorts, such as Cohort A q-psi data 130A, Cohort B q-psi data 130B, and Cohort C Q-PSI data 130C. The Cohort C Q-PSI data 130C is data that is used for the pre-training of a classifier module 140. In that regard, the classifier module 140 may include a Random Forest (RF) classifier that acts as a sensory gateway, which systematically evaluates the complete array of parameters (e.g., data generated by the Q-PSI) against thousands known diagnostic profiles of patient data. The RF classifier is a powerful ensemble machine learning algorithm for classification (and regression) tasks. In the context of the present disclosure, the RF classifier is trained on a large labeled dataset (e.g., in the ECG application, a cohort of patient ECGs with known diagnoses), so that it can provide a quick "first opinion" on what diagnoses might be present. In some embodiments, the RF classifier builds a forest of decision trees and then combines their predictions to improve accuracy, reduce overfitting, and handle noisy or complex data better than individual trees. For each potential diagnosis of a tested case, the RF classifier outputs probability scores, which provides a statistically based differential diagnosis closely mirroring the first step of the decision-making processes of an experienced clinician. This initial differential diagnosis serves as a transparent, auditable foundation upon which the reasoning structure of the present disclosure then acts. It is understood that although the embodiment of Fig. 1 implements a classifier module (e.g., RF classifier) to train the quantitative parameters, other types of modules may also be used to accomplish similar tasks. For example, a support vector machine may be used instead of the RF classifier in alternative embodiments.

Still referring to Fig. 1, the Cohort A q-psi data 130A is fed into hemisphere A of the framework 100, and the Cohort B q-psi data 130B is fed into hemisphere B of the framework 100. Hemisphere A mimics one hemisphere of the human brain and is configured to provide a deterministic diagnosis. Hemisphere B mimics another hemisphere of the human brain and is configured to provide a semantic interpretation.

Hemisphere A comprises quantitative and semantic parameters 150, including but not limited to centroid A, cluster B DX, and gold lattice A. In more detail, for every patient that is in a hemisphere represented in a grid made by the classifier module 140, a gold label diagnosis is generated. In the context of machine learning, gold label refers to ground truth. Here, the gold label serves as part of the training data, and all the gold label diagnoses together form the gold lattice A. Essentially, the gold lattice A comprises a grid where every gold label is represented, and every point in the grid contains information about a particular gold label. The gold label lattices form clusters (e.g., cluster B DX in Fig. 1), and every cluster has a centroid (e.g., centroid A in Fig. 1), which is a center of gravity of the locations of all the points in the cluster in a mathematical sense. Individual clusters are used as batches for training a machine learning model, such as the M1 LLM (discussed in more detail below) in Fig. 1. The batches corresponding to different diagnoses may be trained one after another.

Note that the M1 LLM itself has already been trained (e.g., as a commercially available LLM offered by a third part), and the training discussed herein refers to the skills it will be using in the context of the present disclosure. Essentially, the training herein is with respect to the Retrieval-Augmented Generation using Graphs (Graph-RAGs), which are represented by the two hemispheres A and B in Fig. 1. Graph-RAG is an advanced AI technique that enhances large language models (LLMs) by building and querying knowledge graphs from text. A Graph-RAG pertains to a spatial arrangement of data for retrieval by an LLM and extracts entities and relationships, organizes them into graphs, and uses graph traversal and community summaries for context. Graph-RAGs (or RAGs in general) can reduce LLM hallucinations by grounding LLM responses in real, external data, as well as the use of tools such as Guardrails.ai. RAG combines a retriever (which searches a knowledge base or documents) with a generator (like GPT-4). Instead of answering a prompt from memory alone, the LLM receives relevant retrieved passages as context. RAG grounds responses in real sources, encourages transparency by providing real citations for its answers, and can help minimize fabrication by reducing the need for an LLM model to make up facts. In some embodiments, the RAGs can be structured by a plurality of dimensions (e.g., 17 dimensions) and therefore corresponds to a multi-dimensional space. Each point in this multi-dimensional space represents a different patient with all of its patient properties. Certain groups of patients (e.g., also referred to as tribes) have rules together. The rules are the skills for the LLM. For example, notes (e.g., relating to a diagnosis) may be written about these patients, what are special things the LLM should take into account when a patient is in a related area. When a new patient is examined by the system, the system compares this new patient with all other patients it already had, and the system may apply hundreds of rules to this comparison, which may lead to a final diagnosis that is insightful.

Hemisphere A also comprises a block 160, which may visually represent the summary of the skills that are created, modified, or improved by the training. In more detail, two LLMs are involved: the M1 LLM and the M2 LLM. In the context of the present disclosure, the role of the M1 LLM is the integration of expert-level medical knowledge. Building upon the robust statistical foundation set by the tokenization of extensive medical data that is then processed by the classifier module 140, the framework 100 herein integrates a specialized large language model (i.e., the M1 LLM, also interchangeably referred to as LLM-1) that has been extensively trained on a corpus of medical literature and advanced cardiology guidelines. Unlike black-box AI models, the M1 LLM explicitly incorporates comprehensive clinical knowledge through carefully designed rule cards in a RAG system with prompt texts, enabling nuanced evaluation and refinement of the RF classifier's outputs. By weighing patient-specific contexts, recognized medical patterns, and detailed clinical guidelines, the M1 LLM effectively emulates the reasoning of an expert cardiologist to reduce systematically the differential diagnosis to the final result. Its final diagnostic outputs are deterministic, grounded not merely in raw statistical likelihood but in coherent clinical logic and validated textbook-like rules.

In terms of mimicking a human brain, the M1 LLM serves as short-term memory, as it focuses on real-time analysis of incoming data, processing information in manageable chunks. For example, when analyzing a 10-second ECG segment, this layer identifies immediate patterns such as arrhythmias or supraventricular premature beats, creating detailed representations of the events. In some embodiments, the M1 LLM continuously reprocesses tokens from multiple streams (patient data, ECG, environment sensors, etc.), maintains a dynamic context window, adapts to new tokens in near real-time with minimal computational overhead, and executes immediate reasoning tasks (e.g., interpreting an ECG waveform, controlling a robot's movement).

The M1 LLM also acts as a deterministic diagnostician. It is a domain-specialized language model (smaller and fine-tuned for medical reasoning in the context of the present disclosure) that does not generate free-form answers from scratch, but rather works within a controlled prompt to refine and finalize the diagnosis. In some embodiments, the M1 LLM receives three main inputs (which may be comprised in the Cohort A/B/C data 130A-130C discussed above): (a) the patient's feature vector (the structured data from quantum wave tokenizer), (b) the RF classifier's predicted diagnosis probabilities (which identifies likely diagnoses and their confidence), and (c) a set of relevant Prompt Nodes retrieved from the prompt bank based on the case's feature location. These prompt nodes contain rule-like instructions or contextual cues on how to handle cases in that neighborhood of feature space. For example, a prompt might be: "If both atrial fibrillation and atrial flutter are suspected, remember they are mutually exclusive and check for rhythm regularity to decide between them." Such a prompt would be retrieved for a case in which the RF gave high probabilities to both AFib and atrial flutter, guiding the M1 LLM to apply a specific rule.

Given these inputs, the M1 LLM's task is to produce the final diagnostic decision in a deterministic, rules-based manner. Internally, the M1 LLM can be thought of as executing a sequence of "if-then" checks and threshold comparisons that have been encoded in its prompt (and in its fine-tuned weights). It filters the list of candidate diagnoses from the RF classifier by applying the conditions from the prompt rules and any hard exclusions or requirements defined therein. The outcome is a pruned, refined list of diagnoses that the M1 LLM outputs as the system's answer, often accompanied by structured explanation text. Crucially, the M1 LLM's operation is made deterministic by the structure of the prompt and model constraints: if the same inputs and prompt are given, the M1 LLM will consistently produce the same output list. There is no temperature sampling or open-ended generation in this phase - it is more akin to a rule engine executing, with the M1 LLM ensuring the integration of rules and data is done with the nuance of a clinician. In effect, the M1 LLM bridges the gap between raw statistical prediction and coherent clinical logic. The RF classifier provides the "instinctual" probabilities (like an initial guess), and the M1 LLM, guided by rules, turns that into a final decision that respects medical logic and avoids contradictory outputs.

The M1 LLM's role can also be analogized to an expert system or an "analysis cortex." It explicitly uses medical knowledge (embedded in its training on guidelines and in the prompt rules) to double-check the RF classifier's suggestions. For instance, if the RF classifier suggests two diagnoses that normally never occur together, the M1 LLM can use a prompt rule to eliminate one. If the RF classifier overlooks a subtle clue because it was statistically rare, the M1 LLM may re-insert a diagnosis if a prompt reminds it of that clue. Importantly, the M1 LLM's decision process is auditable. The prompt nodes it used can be logged, and the final output is derived via clearly defined conditions. Thus, a trace may be made as to why a certain diagnosis was made - e.g. "Diagnosis X was output because RF classifier suggested it with probability 0.60 which passed the 0.05 threshold, and none of the exclusion rules for X triggered given the feature values, while diagnosis Y was filtered out due to rule A (as encoded in the prompt) requiring feature Q < 0.5 which was not met." Such detail provides explainability at a level unattainable with a monolithic neural net. In effect, the M1 LLM ensures that the system's outputs are "textbook-like" - grounded in explicit rules and medical rationale, not just correlation. This design directly addresses the earlier challenges of determinism and auditability: M1 LLM's outputs are stable and reproducible, and they conform to known rule-based standards.

Meanwhile, the role of the M2 LLM is the clinical reasoning and interpretative depth. In more detail, in order to further enhance interpretability and clinical depth, a second, higher-order reasoning language model (i.e., the M2 LLM, also interchangeably referred to as LLM-2) engages in sophisticated analysis of the outcomes provided by the M1 LLM. The M2 LLM serves a dual purpose: initially contributing significantly to reinforcement learning and iteratively refining the diagnostic rules, it subsequently provides a nuanced interpretation of the final diagnosis. By reviewing the comprehensive parameter sets alongside the finalized diagnostic labels produced by the M1 LLM, the M2 LLM generates interpretative insights far exceeding a simple diagnostic enumeration, delivering deeper clinical understanding and interactive responsiveness to a user's inquiries. In terms of mimicking a human brain, the M2 LLM serves as long-term memory, as it integrates the outputs of the short-term memory with historical patient data. By aggregating information across multiple recordings- spanning minutes, hours, or even months-this layer builds a comprehensive, personalized view of the patient's condition. This long-term memory ensures that the system doesn't just respond to isolated events but understands how they relate to the patient's broader health context. (Note that additional LLMs may also be employed.) The M2 LLM serves as episodic storage, such summaries of M1 LLM's context window (or other noteworthy events) are stored by the M2 LLM in a retrievable database whenever significant changes occur (e.g., new recordings, major anomalies). The M2 LLM also serves as an expert knowledge database, where it houses domain-specific data (medical guidelines, manufacturing tolerances, robotics algorithms, etc.). The M2 LLM listens for keywords or triggers in M1 LLM's current context and surfaces relevant information. When the M2 LLM detects a match, it retrieves the relevant data or past episodes and provides them to the M1 LLM, enhancing M1 LLM's immediate context.

In the context of the present disclosure, while the M1 LLM and the RF classifier together provide a strong base (covering the majority of routine cases with transparent logic), the M2 LLM's role is to continuously improve and expand the system's reasoning. The M2 LLM can therefore be seen as a meta-learner or "prompt engineer" that observes how the M1 LLM performs and then refines the prompt rules for next time. During training (or periodic retraining phases), cases are processed in batches (grouped by similarity or a focus condition) and M1 LLM's outputs are compared to ground truth labels (e.g., gold labels). Whenever the M1 LLM makes an error - either a miss (false negative, termed A1) or a false alarm (false positive, termed B1) - M2 LLM is triggered to analyze the situation. The M2 LLM is given the full context of the batch: the patient features, what M1 predicted, what the true labels were, and which prompt rules were in play. The M2 LLM's task is not to diagnose patients directly, but to diagnose the reasoning process itself and suggest a minimal fix.

In essence, the M2 LLM performs reinforcement learning on the reasoning rules. It treats the prompt rules as policies that can be adjusted. For example, suppose the M1 LLM frequently misses a particular diagnosis in a cluster of cases because the threshold for a certain feature is too high - the M2 LLM may detect this pattern and suggest lowering that threshold in the prompt rule to catch those cases. Or, if the M1 LLM tends to erroneously include a diagnosis when a particular artifact is present in the data, the M2 LLM may add a new "veto rule" to exclude that diagnosis in such context. The M2 LLM essentially performs a credit assignment on M1 LLM's mistakes: was the issue that a rule was too lax, too strict, or missing entirely? Then it proposes a change accordingly. This proposal is done via its own language modeling capabilities - it can generate a new prompt snippet or modify an existing one and output that as a potential patch.

To ensure stability, these changes are typically constrained to the local cluster of cases (so they don't adversely affect distant scenarios). In practice, the training cases are organized by cluster (using the feature space and RF classifier outcomes to cluster similar situations). The M2 LLM operates on one cluster at a time, fine-tuning the rules for that cluster in a way that improves performance on that batch without harming performance on other batches. Technically, this is implemented by associating prompt rules with cluster centroids (the Prompt Nodes in the graph have coordinates) and by only adjusting the prompt tied to that cluster or creating a new prompt node if needed. Over many training iterations, this results in a prompt bank that is optimized for different regions of the problem space - an emergent episodic memory of how to handle various corner cases.

The M2 LLM's operation mode is analogous to a mentor or supervisor. It has the benefit of hindsight (knowing the gold-standard answer) and it "teaches" the M1 LLM by updating the rules. A key design element is that the M2 LLM's changes are immediately recorded as new Prompt Nodes in the prompt bank (along with metadata like parent prompts and performance deltas). This forms the episodic memory of the system: each Prompt Node can be seen as a memory of a lesson learned - often derived from a specific batch of cases or even a single important case. Over time, the prompt bank grows to cover a wide array of scenarios, including many that were not explicitly in the original training data (since the M2 LLM can interpolate between prompts and create new ones for truly novel situations). The structure is dynamic: if a new diagnosis (label) emerges in the medical field, the M2 LLM can even add a new node expanding the label space to handle it - something practically impossible for a static classifier.

The M2 LLM uses reinforcement signals like improvements in F1-score or reduction in errors to guide its learning. It effectively performs policy gradient updates but in the space of language (prompts) rather than numeric weights. By doing so, it keeps the system's logic aligned with real-world outcomes. Importantly, the M2 LLM's suggestions are typically reviewed or at least constrained by human-written archetypes. The initial prompt bank is seeded with human-crafted corner-case prompts (the "primed prompts"). The M2 LLM does not generate rules out of thin air; it is guided to modify or blend these archetypes, ensuring that the resulting rules remain within the bounds of human plausibility. This combination of human initialization and AI optimization yields a very powerful effect: the system improves with experience, yet remains safe and interpretable. The M2 LLM's continual learning can be run offline on new data (e.g., periodically retraining with the latest cohort of cases), and in the field, the system can also operate in an online learning mode: if a user (e.g., a doctor) corrects it or provides feedback on a case, the system herein can integrate that as a memory update (for instance, marking that case's cluster and triggering a prompt adjustment for it). Thus, the system herein is capable of learning from human feedback via dialogue, updating its rules in a controlled way. This addresses the challenge of incorporating human-in-the-loop refinement without simply fine-tuning black-box weights - instead, the human feedback directly alters a transparent rule or threshold.

In summary, The M2 LLM endows the system herein with self-improvement and episodic memory. It turns every mistake into an opportunity to learn a new rule or tweak an old one, akin to how a doctor learns from cases they got wrong. Over time, the effect is that system's knowledge matures: its initial "genetic" knowledge (from the RF classifier and base prompts) is augmented by an accumulated "experiential" knowledge (the prompt bank grown by the M2 LLM). This dual knowledge base is what allows the system herein to achieve both high accuracy and high explainability - because even its new learnings are stored as explicit rules, it doesn't turn into a black box as it evolves. Instead, it becomes more interpretable, since it can say "I used to think X in this scenario, but after case #123 I learned rule Y, which is why I now conclude Z."

The M1 LLM and M2 LLM collectively form a powerful two-stage reinforcement learning (RL) framework that is capable to continuously adapt and self-improve in the manner as follows:
1. Intuitive Analysis (performed by the M1 LLM):
   The M1 LLM rapidly evaluates patient data against explicitly structured "diagnosis rule cards," each defining thresholds and clinical decision logic. This rapid pattern-recognition mirrors a seasoned clinician's intuitive diagnostic process.
2. Advanced Reasoning Cortex (performed by the M2 LLM):
   The M2 LLM serves as a dynamic reasoning cortex, actively refining the diagnosis rule cards based on comparative feedback between M1 outputs and gold-standard clinical labels. Through iterative loops, the M2 LLM adjusts rule card thresholds, weights, and prompts, thereby progressively aligning the M1 LLM's intuitive outputs with verified clinical realities.

In more plain English terms, the M1 LLM makes a guess and generates an informed decision or statement about a particular patient, which may be done without knowing the gold label. The decision or statement generated by the M1 LLM is then reviewed by the M2 LLM, which does know the gold label. The M2 LLM then modifies the skills (e.g., rules for the LLMs) so that the M1 LLM is capable of making a better guess the next round. In some embodiments, one or more of the rule cards comprise nearest neighbor personalized range information corresponding to the patient, and the initial diagnosis (e.g., initial cardiac diagnosis) is generated by the M1 LLM based on the one or more rule cards that comprise nearest neighbor personalized range information corresponding to the patient. In some other embodiments, one or more of the rule cards comprise personalized thresholds computed on the basis of a local control cohort, and the initial diagnosis (e.g., initial cardiac diagnosis) is generated by the M1 LLM based on the one or more rule cards that comprise personalized thresholds computed on the basis of a local control cohort.

Still referring to Fig. 1, hemisphere B comprises quantitative and semantic parameters 180, including but not limited to centroid B, cluster B DX, and gold lattice B, which are similar to their respective counterparts in the quantitative and semantic parameters 150 discussed above with reference to hemisphere A. Based on the Cohort B q-psi data 130B and the quantitative and semantic parameters 180, the M1 LLM and the M2 LLM can perform a validation loop in the hemisphere B. In more detail, when a certain patient has been trained by changing a rule (e.g., a skill), then the same rule is tested also on patients with similar properties in the Cohort B q-psi data 130B to ensure that no over-fitting occurs. The rule change will be accepted only when Cohort B also profits from the rule change, which indicates that the rule change is objective and not based on a coincidence.

In the context of Fig. 1, the rule change is visually represented by the output A generated by the M2 LLM in block 160. In other words, the M2 LLM generates the output A (representing a rule change) and sends it to hemisphere B for validation. Using one or more GPUs, the rule is validated based on the quantitative and semantic parameters 180 and then sent back to hemisphere A. Thus, one hemisphere is validated with the skills of the other hemisphere to ensure that the data is always supporting the skills that have been trained on the other side. The M2 LLM in hemisphere B is also configured to generate an output B that represents an inference produced at an inference stage.

Note that the M1 and M2 LLMs in hemisphere A may not be completely identical to the M1 and M2 LLMs in hemisphere B, as that may constitute a waste of resources. Instead, the M1 and M2 LLMs may be trained in slightly different modes in hemisphere A and hemisphere B. For example, the M1 LLM in hemisphere A is supposed to always make a direct diagnosis. As such, no other speculation should be made about the patient. Meanwhile, the M1 LLM in hemisphere B is allowed to make a speculation about the patient's health. Both the M1 LLM in hemisphere A and in hemisphere B may be used together to generate a prediction in the inference stage.

In some embodiments, the system of the present disclosure can have two modes of operation: deterministic mode and episodic learning mode. In the deterministic mode (which is the normal inference mode when deployed), the system herein behaves like a fixed-function deterministic expert. A given input case goes through the Q-PSI -> RF classifier -> prompt retrieval -> the M1 LLM, producing a diagnosis with full traceability. In this mode, there is no randomness; the outputs are reproducible and the reasoning follows the fixed prompt rules and RF classifier thresholds. This is what would be used in a live clinical setting or any production environment where consistency and reliability are paramount. The system herein will also provide explanations (via M1 LLM's structured output or the M2 LLM acting as a "narrator" model to explain the applied rules) to the end-user, thereby functioning as a deterministic and auditable AI assistant.

In the episodic learning mode (typically engaged during training cycles or updates), the system herein temporarily allows the adaptive loop with the M2 LLM to run and update the prompt bank. This mode is more experimental and involves trying prompt adjustments and evaluating their impact on a validation set (or receiving human feedback). One can consider this mode as "writing to memory", whereas the deterministic mode is "reading from memory." The separation of these two modes ensures that when the system herein is being used for real decisions, it is not simultaneously trying to learn and change behavior on the fly (which could be risky). Instead, learning happens in a controlled way, and once new rules are accepted, they become part of the fixed memory for future inference. This design is akin to having a training phase and an inference phase, but in the system herein, the training phase is not about tweaking millions of weights with gradient descent, but about editing a knowledge graph of prompts. This is much more transparent and modular. If something ever goes wrong (say a rule makes things worse), it can be quickly identified and rolled back, unlike a mysterious weight update buried in a neural network.

This dual-mode capability makes the system herein highly flexible in deployment. For a highly regulated domain, one might lock the system in deterministic mode and only allow learning mode offline with oversight - ensuring all changes are audited and validated. In less critical deployments, the system herein could learn on the fly from interactions (with maybe a supervisor confirming each rule change). In all cases, because learning results in explicit rule changes, any update can be examined, tested, or even manually edited by developers or experts. This addresses a common concern with AI in sensitive fields: the inability to fine-tune or correct the system without retraining. In the system herein, if a regulator or hospital says "we don't like this particular rule or we have a new guideline to incorporate," one can directly insert or modify a Prompt Node representing

that rule. The system is user-controllable at the knowledge level, a far cry from black-box models where one can only hope the training data induces the desired behavior.

To summarize, the system herein operates either as a stable reasoning engine (when used for decisions, it's fully deterministic and uses its current memory) or as a learning framework (when updating its memory based on new cases or feedback). This clear delineation and the interpretability of the memory updates ensure both safety and adaptability. It is a novel approach that merges the benefits of static expert systems (predictability) with the benefits of learning systems (continuous improvement).

In some embodiments, the M1 LLM and/or the M2 LLM are implemented using the following hardware components:

| Hardware | Purpose |
|---|---|
| High-End PC (RTX 4090, Ryzen 9 7950X3D, 64GB RAM, 2TB NVMe SSD, 1000W PSU, Windows 11 Pro) | Fine-tune LLM-1 (10s ECGs), early model training |
| NVIDIA GB10 DIGITS (1 PFLOP AI Compute, 128GB Unified Memory) | Faster fine-tuning & HPO (~15 min per epoch), dual-LLM training |
| Custom AI ECG Device (based on GB10 DIGITS or ASIC future implementation) | Final AI deployment in real-world ECG machines |

In an example embodiment, the M1 LLM, as a short-term analysis model, analyzes 10 second ECG chunks (e.g., with ~40,000 labeled examples). Meanwhile, the M2 LLM, as a long-term aggregation model, aggregates multiple 10 second analyses (e.g., 6x10s= 1 minute) and detects slow P-wave and rhythm changes. In some embodiments, the two phases of LLM training may be performed as follows:
Phase 1: Training the M1 LLM (10s ECG Analysis)
   - Purpose: Train a first AI model to analyze 10 seconds ECG chunks.
   - Training Data: 40,000 labeled ECGs (10 seconds each).
   - Hardware: RTX 4090 (~1.5 hours per epoch).
Phase 2: Training the M2 LLM (Long-Term Aggregation)
   - Purpose: Train a second AI model to process longer ECG recordings (1-3 minutes).
   - Training Data: 100 clinical trial patients (1-3 min ECGs, split into 6× 10s chunks per patient).
   - Hardware: GB10 DIGITS (~15 min per epoch).

In some embodiments, the fine-tuning of the LLMs (as AI models of the present disclosure) here can be done in two phases:
- Initial Training on RTX 4090. For training a single LLM, about 1.5 hours may be needed per epoch in some embodiments. For training a dual LLM, about 3 hours may be needed per epoch in some embodiments.
- Optimized Training & HPO on GB10 DIGITS. For training a single LLM, about 15-20 minutes may be needed per epoch in some embodiments. For training a dual LLM, about 30 minutes may be needed per epoch in some embodiments.

In some embodiments, the following AI fine-tuning roadmap may be used to implement the LLMs of the present disclosure:

| Task | Data | Hardware |
|---|---|---|
| Fine-tune LLaMA 3.23B (LLM-1, 10s ECG only) | 40,000 labeled ECGs (10s each) | RTX 4090 |
| Validate LLM-1 on real test ECGs (10s chunks only) | Test dataset with real-world ECGs | RTX 4090 |
| Fine-tune LLaMA 3.23B (LLM-2, 1-minute aggregation) | 100 patients with 1-3 min ECGs | GB10 DIGIT S |
| Train LLM-1 + LLM-2 to collaborate in real-time | Multi-LLM fine-tuning with long-term storage | GB10 DIGIT S |
| Deploy AI ECG system on real hardware | Integrated AI-powered ECG system | GB10 DIGITS / ASIC |

In some embodiments, the LLM deployment and real-time inference may be implemented as follows:

| Task | LLM Role | Hardware |
|---|---|---|
| Deploy AI ECG machine with GB10 DIGITS | Real-time inference | GB10 DIGITS |
| Test clinical integration of AI ECG | M1 LLM (10s analysis) + M2 LLM (long term patterns) | ECG Machine with AI |
| ASIC Hardware optimization | Compact AI ECG inference | Future AI ASIC chip |

In some embodiments, a retrievable memory & query/storage manager may be optionally implemented. For example, it may include a dedicated software or hardware module that indexes, compresses, and stores M1 LLM's context snapshots. It also provides fast retrieval of domain knowledge or episodic data based on M2 LLM's requests. In some embodiments, parallel hardware acceleration may be used to implement various aspects of the dual LLM scheme discussed above. For example, analog ASICs, GPUs, or other specialized processors can be used to handle the repeated, loop-based reprocessing of tokens without large-scale matrix storage. The parallel hardware acceleration minimizes latency, enabling continuous or high-frequency data analysis in real- world scenarios (e.g., continuous Holter ECG or real-time robotics).

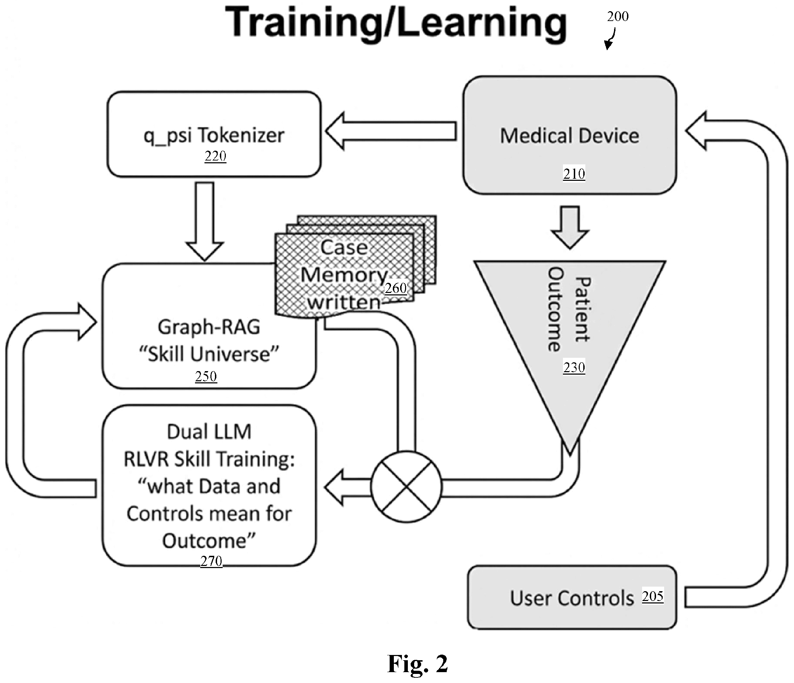

Figs. 2-4 illustrate process flows involving the various components discussed above with reference to Fig. 1 according to embodiments of the present disclosure. For reasons of consistency and clarity, similar components appearing in Figs. 2-4 will be labeled the same. Referring to Fig. 2, a training/learning process flow 200 is illustrated. As a first step of the process flow 200, based on user controls 205 (e.g., a hardware-based or software-based user interface), a medical device 210 produces raw medical data. The medical device 210 may include, but it not limited to: electrocardiogram machines (ECG machines) configured to generate electrocardiograms (ECGs) from heart electrical signals, electroencephalogram machines (EEG machines) configured to generate electrocardiograms (EEGs) from brain electrical signals, ultrasound systems and devices configured to generate/transmit and receive high-frequency sound waves or signals, magnetic resonance imaging (MRI) systems and devices configured to generate and receive radiofrequency (RF) signals, X-ray systems and devices configured to capture emitted X-ray beams and generate visual representations of target portions of patients' bodies, and computed tomography (CT) or computerized axial tomography (CAT) scan devices and systems configured to capture emitted X-ray beams and generate visual representations of target portions of patients' bodies. In some embodiments, the medical device 210 may comprise, or may be coupled to, the wireless communications device 110 of Fig. 1.

The raw medical data generated by the medical device 210 is sent to a Q-PSI tokenizer 220 (i.e., the Quantum Wave Tokenizer). As discussed above with reference to Fig. 1, the Q-PSI tokenizer 220 is configured to convert the raw medical data from the medical device 210 into precise, processable, understandable, clinically interpretable parameters. The manner in which such a conversion process (e.g., fitting the raw medical data with quantum waves and subsequently processing them with an interpreter) takes place will be discussed in more detail below with reference to Fig. 5.

In addition to outputting the raw medical data to the Q-PSI tokenizer 220, the medical device210 also produces data pertaining to a patient outcome 230. The patient outcome 230 may indicate how well a particular patient fared over the course of time, but it may or may not be known immediately. For example, the patient outcome 230 may indicate that the patient has been healthy for the last two years after the raw medical data has been measured, or that the patient had a heart attack three months after the raw medical data has been measured, or that the patient has had elevated blood pressure (e.g., above a predefined threshold) over a certain period of time. In some cases, the raw medical data taken from the same patient will necessarily vary significantly over time, for example, due to a medical procedure having been performed (e.g., ablation). Regardless, the patient outcome 230 is not binary but may include a plurality (e.g., hundreds or even thousands) of different outcomes corresponding to different diagnoses. All of these different outcomes and/or diagnoses may be used retroactively to train the system herein.

The tokenized data generated by the Q-PSI tokenizer 220 is sent to a Graph-RAG 250, also referred to as "skill universe" herein. The Graph-RAG 250 is represented by the hemispheres A and B and the skills discussed above with reference to Fig. 1. As such, the Graph-RAG 250 includes the memory that contains the processed (e.g., tokenized) medical data, as well as the skills (e.g., rules for the LLMs) that are trained. The memory and the skills collectively form the knowledge of the system. Note that the Graph-RAG 250 may also comprise case memory 260, which refers to the data pertaining to all the patients that has been collected by the system during training and during episodic learning. The case memory 260 may continue to increase with every new patient on which the system is trained.

In some embodiments, the setup of the Graph-RAG 250 comprises a vector database of prompt nodes that each contain a piece of reasoning logic. The top relevant snippets of the prompt nodes are retrieved and fed into an LLM for answer generation. Such a retrieval may be done via a Ball Tree or KNN search in the feature space. This forms effectively a graph of knowledge, where the connectivity is implicit in the distance metric of the feature space and explicit through parent-child relationships of prompts (when the M2 LLM creates a new prompt, it records which prior prompts influenced it). Over time, as the M2 LLM adds prompts, this becomes a growing graph, one that reflects the topology of the problem domain as experienced by the model. For example, prompts that deal with arrhythmias may form one cluster, while prompts for ECG artifact handling form another cluster. If a case lies between clusters, the prompts from both cluster may be retrieved, effectively blending knowledge. If no cluster is a good fit for the case, the M2 LLM flags it as a truly novel scenario for which a new node may be needed.

This graph structure is inherently explainable. A user or auditor can inspect the prompt nodes connected to a given diagnosis outcome, see their content (which is usually in natural language or pseudocode form describing the rule), and understand the rationale. The graph also provides transparent updates: every time a new prompt node is added by the M2 LLM, it is akin to adding a new entry in a changelog or knowledge base. The node carries metadata of when it was added, why (which errors it was addressing), and links to predecessor nodes (the parent _ids) if it was derived from tweaking existing rules. This is vastly more transparent than weight updates in a neural network. It means the evolution of the model is trackable - one can see versioned changes in the prompt bank and even revert or simulate different versions of the reasoning if needed. For instance, for regulatory review, one could produce a difference of the knowledge graph before and after a batch training to show what exactly changed in the system's reasoning.

Another benefit of the graph RAG approach is that it enables modularity. If for some reason a certain set of rules (say all prompts related to pediatric cases) need to be revised or isolated (e.g., due to a new guideline), they form a subgraph that can be updated without overhauling the entire model. The rest of the system remains untouched. Traditional end-to-end models lack this granularity - changing the model for one subset of cases can unpredictably affect others. The knowledge graph herein is much more surgical in that respect. It is feasible to maintain different branches of the prompt graph for different institutions or user preferences as well. For example, a certain hospital might customize a few prompt nodes to align with their protocol (e.g., how aggressive to be in diagnosing a borderline condition), and those would live as small modifications in their copy of the graph, while still benefiting from the overall architecture.

From a technical perspective, the graph acts as a memory cache that an LLM (e.g., the M2 LLM) uses to ground its reasoning. Unlike typical RAGs where the LLM might still generate a lot of free text around retrieved info (and potentially hallucinate), in the system herein the retrieved prompt text effectively constrains the LLM's output. The M1 LLM is not merely handed some reference text. Rather, the M1 LLM is being handed instructions and even templated language that it should follow. This is a key distinction - the prompts are active constraints, not passive context. They ensure that the M1 LLM's output adheres to known good patterns. In many cases, the M1 LLM's final answer is templated by the prompt (with slight variations for patient data), which is why it can be deterministic and audited. Essentially, the system herein uses retrieval to inject domain knowledge and guardrails into the generation process in a structured way.

In summary, the structure of the Graph-RAG 250 herein is what allows the system to be explainable by design and updatable by design. It stores knowledge in a human-comprehensible form (nodes with rules) and organizes that knowledge in a spatial layout that corresponds to real-case variations, making retrieval relevant. Every piece of reasoning has a place in that graph. When the system herein generates an explanation, it effectively walks a user through the graph: "the case was routed to cluster A, which triggered rules 1, 2, 3, leading to diagnosis X." When the system learns, it is editing the graph: "added rule 4 under cluster A to handle new observation." This graph paradigm is at the core of how the system herein is capable of delivering trustworthy AI - it is not only about getting predictions right, but about structurally encoding and communicating the why behind those predictions.

The data from the Graph-RAG 250 and the patient outcome 230 are collectively sent to perform Dual LLM RLVR (Reinforcement Learning with Verifiable Rewards) Skill Training 270, which indicates what data and controls mean for outcome. The dual LLMs refer to the M1 LLM and the M2 LLM discussed above with reference to Fig. 1. RLVR is a particular type of reinforcement technique that is not just based on the so-called policies. In that regard, a policy means that the system estimates whether the learning is good or bad, and this estimation is done on some noise parameters, on some parametric fits, and/or on some vectorization of outcomes. Although the policy-based learning involves complex formulas, it may not be sufficiently powerful, because sometimes it makes the wrong decision. In addition, if the policy-based learning occurs in the feature space of an LLM, it may be a complete black box. In contrast, RLVR improves LLM reasoning capabilities by using automated and objective feedback (which may be binary)-such as code tests or math solvers-to verify correctness, removing the need for human annotation. In some embodiments, RLVR may check with every step whether the outcome is better classified by the system after a particular change of the skill. The outcome may refer to the matching between the gold label and the prediction.

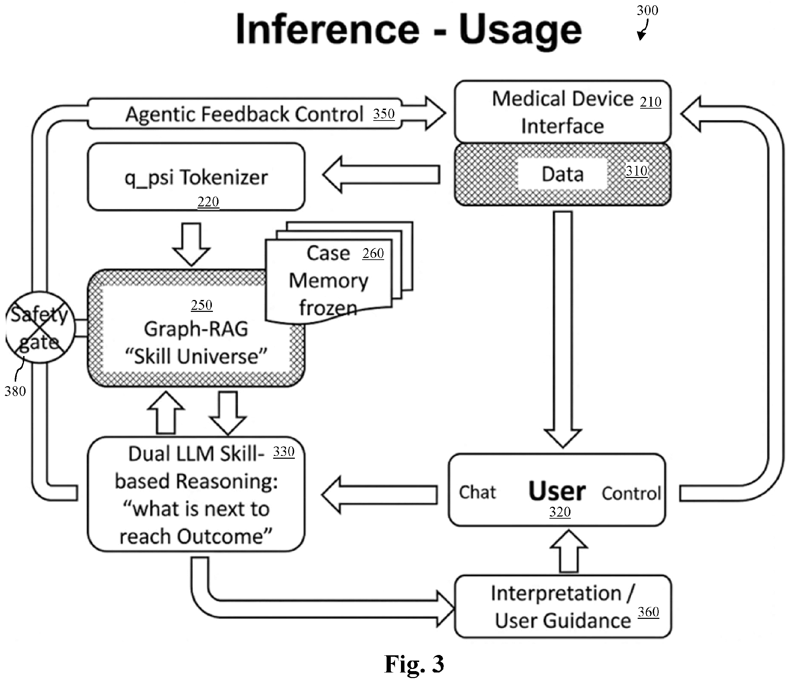

Referring now to Fig. 3, an inference-usage process flow 300 is illustrated. Again, similar components that were discussed above in association with Fig. 2 are labeled the same in Fig. 3. In the context of Fig. 3, inference refers to the output generated by the agentic LLMs of the system herein for a single case (e.g., a particular patient). The inference is not limited to just a diagnosis, but it may include a suggestion or recommendation on one or more actions that should be performed. For example, suppose that a medical device is an ablation tool, the inference made by the agentic LLMs may indicate that there has been enough ablation, and that the ablation should stop. As another example, in a diagnostic scenario and/or in an emergency care situation, the agentic LLM may suggest a certain sequence of diagnostic measures because the patient has certain known risks factors.

A properly generated inference herein involves an interaction between a user (e.g., a medical professional) and the medical device 210 and between the user and the dual LLMs discussed above. For example, a user 320 may control (e.g., by issuing commands) the medical device 210 via an interface of the medical device 210. The medical device 210 generates raw patient data 310 (e.g., ECG data) that is sent to both the user 320 (to be reviewed by the user) and the Q-PSI tokenizer 220 (to be converted into tokens). The user 320 may also communicate with the dual LLMs via a chat-based user interface. Based on the chat input received from the user 320, the dual LLMs (e.g., the M1 LLM and the M2 LLM discussed above) perform LLM skilled-based reasoning 330, including what is next to reach outcome. The dual LLMs communicate with the Graph-RAG 250 ("Skill Universe) back and forth to perform the skill-based reasoning. Note that the case memory 260 is frozen in the process flow 300, meaning that the rules remain unchanged.

The dual LLM skill-based reasoning 330 generates an output (e.g., an inference) that is then fed back as a part of agentic feedback control 350 to the medical device 210. The medical device 210 may adjust its operations at least in part based on the agentic feedback control 350. The output of the dual LLM skill-based reasoning 330 is also fed back to the user 320 as a part of interpretation/user guidance 360, which may include not just diagnoses, but also suggestions or recommendations on what action(s) to perform next. The user 320 may then adjust the control of the medical device 210 and/or the chatting with the agentic LLMs accordingly. As such, instead of having a reinforcement learning cycle (as was the case in the process flow 200 of Fig. 2), the process flow 300 involves a chat cycle involving the user 320 and the agentic LLMs.

In some embodiments, a safety gate mechanism 380 is implemented as a part of the process flow 300. In that regard, since the agentic feedback control 350 is configured to interact with the medical device 210 directly, a certain level of risk may be introduced by such an interaction. For example, the agentic feedback control 350 may occasionally generate commands for operating the medical device 210 in an undesirable manner (e.g., stopping or disabling a function of the medical device 210, or instructing the medical device 210 to operate in a manner that may be unsafe or at least unpleasant for the patient). To prevent or mitigate the occurrence of such circumstances, the safety gate mechanism 380 (which may include a hardware component and/or a software component) is implemented to provide a check on the agentic feedback control 350. In some embodiments, the safety gate mechanism 380 may comprise various safety rules that may be triggered in response to certain types of content of the output generated by the dual LLM skill-based reasoning 330, such as instructions to operate the medical device 210 beyond certain predefined thresholds. In this manner, the agentic feedback control 350 may be kept in check, and the medical device 210 may be operated safely and securely.

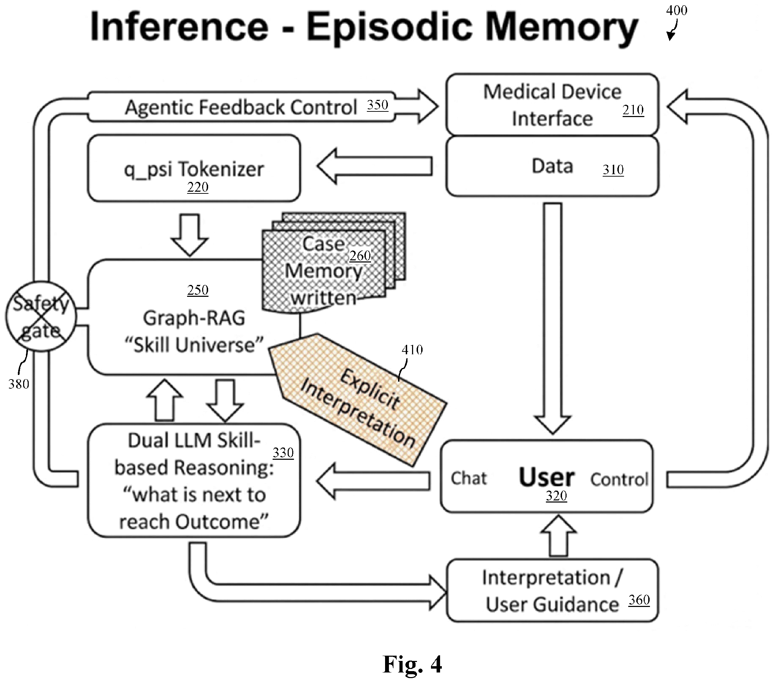

Referring now to Fig. 4, an inference-episodic memory process flow 400 is illustrated. Again, similar components that were discussed above in association with Figs. 3-4 are labeled the same in Fig. 4. The process flow 400 is similar to the process flow 300 of Fig. 3 in many respects. One difference between the process flow 400 and the process flow 300, however, is that the case memory 260 is no longer frozen, but it can be written thereto. This is done to enable or facilitate further training or adjustment.

Another difference between the process flow 400 and the process flow 300 is that the process flow 400 includes an explicit interpretation 410. The explicit interpretation 410 may comprise explicit instructions from the user 320 to tell the agentic LLMs to make a specific diagnosis, or refrain from making a specific diagnosis. Such an explicit interpretation 410 may be written into the Graph-RAG 250 as a skill or a rule. In some embodiments, the explicit interpretation 410 is provided via an interpretation module that is implemented as a part of the chat between the user 320 and the agentic LLMs. The user 320 would have to specifically activate the interpretation module to provide the explicit interpretation 410. This prevents an inadvertent phrase or sentence uttered by the user 320 via the chat from being misinterpreted as an explicit interpretation 410. In other words, the user 320 has to demonstrate specific intent for the explicit interpretation 410 to be construed as such.

In summary, the dual LLM scheme herein dynamically re-ingests new data tokens (e.g., corresponding to (ECG signals, sensor readings, textual instructions, etc.) into at least one specialized LLM (e.g., M1 LLM). This allows the active context to remain accurate and up to date for immediate decision-making. In a long-term memory management context, a second LLM (e.g., M2 LLM) oversees episodic memory storage in a retrievable database. Summaries of the context window are stored when significant changes occur (e.g., new test results, additional patient info, or novel sensor data). The M2 LLM can also be configured to manage an expert knowledge database, continuously monitoring for keywords that trigger the retrieval of domain-specific facts, guidelines, or prior episodes. When relevant terms, anomalies, or user queries appear in the real-time data, the M2 LLM fetches the appropriate background information or historical episodes. This knowledge is then injected into the active context, augmenting M1 LLM's decision-making and inference with richer domain expertise-akin to consulting a medical knowledge base or robotics manual on the fly.

In some embodiments, the operational flow of the dual LLM scheme herein undergoes the following stages:
**1. Initialization**
   ∘ M2 LLM retrieves relevant patient history (in a medical context) or stored knowledge from prior episodes.
   ∘ M1 LLM loads its baseline context window with current input (e.g., "new ECG session started" or "robot initial position established").
**2. Continuous Token Reprocessing**
   ∘ M1 LLM ingests new tokens (sensor data, medical signals, etc.) in streaming fashion.
   ∘ M1 LLM updates the context on the fly and generates immediate outputs (decisions, interpretations, or commands).
**3. Keyword/Trigger Detection**
   ∘ As M1 LLM interprets the data, M2 LLM monitors token streams for relevant triggers-patient risk flags, device anomalies, domain-specific jargon.
   ∘ On detecting a trigger, M2 LLM fetches associated knowledge (e.g., "ventricular tachycardia guidelines," "robotic obstacle avoidance procedure," or stored episodes from the last session) and injects it into M1 LLM's context.
**4. Episodic Summarization and Storage**
   ∘ When M1 LLM transitions to a new activity (e.g., finishing a procedure, discovering
      a critical anomaly), M2 LLM compiles a summary of M1 LLM's context.
   ∘ The system stores this summary (an "episode") in the retrievable memory for future reference.
**5. Output**
   ∘ M1 LLM finalizes the integrated reasoning, potentially returning a diagnosis
      (medical) or an action plan (robotics).
   ∘ M2 LLM stands ready to record that output as part of the evolving episodic log and to recall it if needed in the future.

As discussed above, the dual-LLM scheme herein is one of the unique characteristics of the present disclosure and offers advantages over traditional AI medical systems. The M1 LLM handles real-time reasoning and immediate context interpretation, while the M2 LLM manages structured knowledge retrieval and storage. In addition, whereas traditional AI medical systems often embed knowledge into opaque memory that is not human-readable (and therefore impossible to verify accuracy), the system herein stores long-term information in human-readable ASCII text, allowing explicit review, correction, and structured updates. In some embodiments, the system herein utilizes structured memory storage (e.g., formatted text, knowledge graphs), ensuring all stored knowledge can be audited and edited when necessary. This feature is particularly critical in applications such as medical AI, legal AI, and high-stakes decision-making systems.

The system herein is also scalable and modular with multiple memory units. In more detail, whereas traditional medical AI systems typically employ a monolithic model, the system herein distributes processing across multiple AI cores, allowing for parallelized, scalable computation. Each independent ASIC-accelerated AI unit can specialize in different types of knowledge (e.g., medical, mathematical, or legal memory units). This modular approach enables dynamic scaling, thereby enhancing the adaptability of the system herein.

Furthermore, the system herein improves storing memory based on a "surprise" function (high-gradient tokens are stored automatically) by allowing a secondary validation step, where stored knowledge can be:
- Automatically accepted (for general knowledge).
- Flagged for expert review (in specialized fields like medicine or law).
- Deleted or corrected when necessary.
This prevents the accumulation of misleading or biased information over time.

A mathematical framework usable to implement the dual LLMs discussed above is provided below:
**1. Notation and Preliminaries**
We define:
- $x ∈ {ℝ}^{N \times {d}_{in}}$ as the input sequence with *N* tokens and embedding dimension *d*ᵢₙ.
   *- M* as the structured long-term memory storage (human-readable ASCII-based memory).
- *L*₁ as the Short-Term Memory LLM for real-time transformer processing (e.g., the M1 LLM).
- *L*₂ as the Long-Term Memory LLM for structured knowledge retrieval and memory management (e.g., the M2 LLM).
- *Q*, *K,* Vas the standard query, key, and value matrices in self-attention.
   - 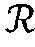 (*q*) as the retrieval function, which retrieves past knowledge based on a query embedding q.
**2. Processing Flow: How the dual-LLM architecture Interacts with Memory**
At each timestep t, the system follows this sequence:
1. Short-Term Processing with LLM-1 (Transformer for Immediate Context Analysis) ${Q}_{t} = {x}_{t} {W}_{Q} , {K}_{t} = {x}_{t} {W}_{K} , {V}_{t} = {x}_{t} {W}_{V}$ ${y}_{t} = {\sum }_{j = {1}^{t}} softmax \left({Q}_{t} {K}_{{j}^{T}}/√{d}_{in}\right) {V}_{j}$ where *yₜ* is the standard transformer attention output for immediate context processing.
2. Querying Long-Term Memory with LLM-2
   ∘ Instead of relying solely on the local context, the dual LLM architecture retrieves stored knowledge from structured memory *M*:
      *qₜ = f*(*Qₜ*) (query function projecting queries to memory space)
      *mₜ =* 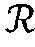 (*qₜ, M*) (retrieval from structured memory) where:
   ∘ *qₜ* represents the semantic embedding of the current token query.
   ∘ 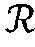 (*qₜ, M*) retrieves stored contextual knowledge related to *qₜ*, ensuring structured recall.
3. Memory-Augmented Context Representation
   ∘ The retrieved knowledge *mₜ* is concatenated with the token embeddings before attention computation: ${{x}^{˜}}_{t} = \left[{x}_{t}\left‖{m}_{t}\right.\right]$
   ∘ This extends the effective context, allowing the dual LLM architecture to process both short-term reasoning and past experiences simultaneously.
4. Memory Storage Update (Surprise-Based Learning)
   ∘ If *qₜ* represents new, surprising information, the dual LLM architecture updates its structured memory *M*: ${M}_{t + 1} = {λM}_{t} + \left(1-λ\right) ⋅ g \left({x}_{t}\right)$ where:
      ∘ *g*(*xₜ*) is the knowledge representation function that encodes new knowledge.
      ∘ *λ* is a forgetting factor, ensuring outdated knowledge is replaced over time.
      ∘ Unlike Titans (which stores embeddings), the dual LLM architecture updates memory as structured, human-readable knowledge.
**3. Mathematical Summary**

| Feature | Mathematical Model of Dual LLM architecture |
|---|---|
| Query Encoding | *qₜ = f*(*Qₜ*) (transformer query projection for structured memory lookup) |
| Memory Representation | *Mₜ* is structured ASCII-text memory with retrieval functions |
| Retrieval Mechanism | *mₜ =* 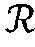 (*qₜ, M*) (structured memory retrieval) |
| Attention Update | *x̃ₜ =* [*xₜ* ∥ *mₜ*] (same principle but interpretable storage) |
| Memory Learning | Surprise-based structured text updates |
| Memory Update Rule | *M*_{*t*+1} = *λMₜ* + (1 - *λ*) *· g*(*xₜ*) |

Mathematically, the dual LLM architecture above extends transformers with an additional retrieval function. The dual LLM architecture also manages structured, verifiable knowledge.

As discussed above, raw medical data (e.g., ECG data, ultrasound data, X-ray data, MRI data, CT data, or CAT scan data) may be converted to tokens via quantum waves according to various aspects of the present disclosure. Currently, interpreting medical imaging data (e.g., ultrasound images) automatically and accurately is a significant challenge due to the complexity of the data and its dependence on the operator's technique. However, with the quantum wave function tokenization and LLM-powered analysis of the present disclosure, medical analysis based on medical imaging data could be transformed. For example, by applying quantum wave analysis, the system of the present disclosure can break down ultrasound or other images into wave-based tokens, providing real-time insights into tissue structures, organ sizes, and spatial relationships. Additionally, in an ultrasound application, for example, the technology can be configured to integrate real-time data from the position and angle of the ultrasound head, allowing the system to "understand" the imaging process as it happens. This would enable unprecedented precision and speed in ultrasound interpretation, creating a truly smart, adaptive imaging tool. The potential impact spans across applications like prenatal care, cardiology, and emergency medicine. Finally, the broader medical device market-including neuroimaging, EEG, and patient monitoring-presents additional applications.

For example, the present disclosure unifies three cutting-edge application domains:
- P-Wave Analytics for Atrial Fibrillation (AF) Recurrence Prediction
- T-Wave Analytics for Ventricular Arrhythmia Risk Assessment
- Predictive ECG Monitoring in Post-Myocardial Infarction Patients

With respect to the application domain of P-Wave Analytics for Atrial Fibrillation (AF) Recurrence Prediction, atrial fibrillation remains a major challenge, particularly after pulmonary vein isolation (PVI), with up to 30% of patients experiencing recurrence due to ectopic triggers outside the pulmonary veins. Detailed P-wave analysis may be performed using several complementary approaches:
- P-Wave Duration (PWD):
   Prolonged PWD (typically >130-160 ms) is indicative of atrial conduction abnormalities and structural heterogeneity. Extended PWD can be correlated with re-entry circuit predisposition and areas of slow conduction.
- Beat-to-Beat (B2B) P-Wave Analysis:
   Rather than relying solely on static measurements, B2B analysis examines the temporal variability in P-wave morphology. A higher B2B index (e.g., >0.5) suggests evolving conduction pathways and multifocal ectopic activity, correlating with increased arrhythmia vulnerability.
- P-Wave Alternans (PWA):
   Microvolt-level amplitude fluctuations across beats (PWA) have been linked to electrophysiological instability. Elevated alternans values (e.g., >4.0 µV in select leads) serve as reliable markers of AF recurrence risk.
- Notched P-Waves and Amplitude Dispersion:
   The presence of M-shaped or "notched" P-waves-often observed in inferior leads- signals conduction delays and asynchronous atrial activation. Complementary metrics, such as the Amplitude Dispersion Index (ADI) derived from wavelet transformations or butterfly plots, capture spatial heterogeneity across leads, further refining recurrence prediction.
- Site-of-Origin Analysis:
   Advanced body surface potential mapping (BSPM) combined with machine learning has enhanced our ability to localize ectopic foci. Simulated integral maps can cluster these foci into distinct atrial regions, guiding more precise interventions.

With respect to the application domain of T-Wave Analytics for Ventricular Arrhythmia Risk Assessment, subtle modifications in T-wave morphology reflect changes in repolarization dynamics-critical for identifying patients at risk of malignant arrhythmias and sudden cardiac death (SCD):
- T-Wave Alternans (TWA):
   Defined as beat-to-beat fluctuations in T-wave morphology, TWA is a marker of arrhythmogenic vulnerability. Elevated TWA levels have been correlated with post-infarction electrical instability.
- T-Wave Angle Variability:
   Variability in the T-wave's angular orientation has emerged as an independent predictor of mortality. Increased repolarization angle dispersion signals an early warning of electrical instability.
- Periodic Repolarization Dynamics (PRD):
   PRD quantifies low-frequency oscillations in cardiac repolarization. In post-myocardial infarction patients, higher PRD values are significantly associated with both sudden and non-sudden cardiac death.
- QT Interval and T-Wave Heterogeneity:
   Prolonged QT intervals and increased beat-to-beat T-wave variability have been linked to a higher risk of malignant arrhythmias, emphasizing the importance of comprehensive repolarization analysis.

With respect to the application domain of Predictive ECG Monitoring in Post-Myocardial Infarction Patients, ECG markers continue to play a pivotal role in prognosticating outcomes in post-myocardial infarction patients:
- Q-Waves, ST-Elevation, and ECG-Derived Markers:
   Traditional parameters-including Q-waves and ST-segment elevations-correlate with infarct size and adverse outcomes. Moreover, indices such as QRS duration, T-wave alternans, and repolarization markers are instrumental in assessing left ventricular dysfunction.
- Dynamic Risk Stratification via AI:
   Recent advancements have shown that AI-powered ECG models can detect subtle signal variations imperceptible to human observers. Deep neural networks can achieve high accuracy in predicting one-year mortality and identifying patients at risk for heart failure. These approaches underscore the potential of integrated, high-resolution monitoring to drive personalized therapy decisions regarding drug regimens, lifestyle, and nutritional modifications.

While CNN (convolutional neural network)-based AI and traditional ECG analytics have each provided valuable contributions to cardiac diagnostics, they have generally been deployed in isolation. Current systems face several critical limitations:
- Fragmented Analysis by Lead or Parameter:
   Many devices analyze P-waves, T-waves, or other markers in isolation, failing to capture the interdependencies among these features. This piecemeal approach overlooks the full interplay of morphological, spatial, and temporal information inherent in the ECG.
- Limited Temporal Context:
   Conventional CNNs excel at recognizing patterns in short time windows-typically seconds-but they lack the capacity to integrate recordings spanning multiple minutes.
- Restricted Data Representation:
   Many existing methods rely on fixed signal transformations (e.g., wavelets) that do not adequately capture the multidimensional complexity of the ECG, potentially missing critical inter-beat and interlead relationships.
- Reliance on Cloud-Based Scientific Platforms:
   Most state-of-the-art systems are available only as cloud-hosted scientific platforms. This model limits accessibility in clinical settings where real-time, on-device analysis is required, and raises concerns about data security, latency, and integration into everyday patient care.
- Data Volume and Training Constraints:
   CNN-based systems typically require tens of thousands of training samples-often derived from short (10-second) ECG snippets. This requirement far exceeds the scale of most clinical trials, where patient cohorts often number only in the hundreds and recordings span longer durations (exceeding one minute). Consequently, CNN-based models struggle to generalize effectively on clinically representative datasets.
- Limited Scalability for Outcome-Based Learning:
   Even model that employ standard classification machine learning on ECG shapes from thousands of patients does not integrate patient-specific outcome data with broader scientific insights. In contrast, the innovative approach of the present disclosure-integrating the intelligent, brain-inspired dual LLM system- forms an internal model that leverages both patient outcomes and established scientific knowledge. This model requires only fine-tuning of hyperparameters, making it viable even on a much smaller cohort of hundreds of patients.
Together, these limitations underscore the need for a solution that seamlessly integrates full- spectrum ECG data, operates on-device (or with minimal cloud dependency), and learns effectively from realistic clinical datasets.

To address the problems discussed above, the present disclosure implements a novel quantum wave function tokenization scheme, which may be integrated with the dual LLM architecture discussed above to achieve advanced ECG analytics to overcome long-standing limitations in signal integration and real-time predictive performance-all while also providing conventional ECG measurements for seamless clinical integration. In more detail, the quantum wave function tokenization scheme herein transforms raw ECG signals into a set of structured, multidimensional tokens. Each token captures critical parameters-amplitude, phase (angle), frequency (period), and time domain-offering a highly granular description of cardiac electrical activity. For example, by encoding multi-lead ECG data as a three-dimensional (3D) vector whose precession over time is modeled as a sum of quantum wave functions, the present disclosure naturally delineates clinically meaningful features such as P-waves, QRS complexes, and T-waves. This approach provides:
i. Precision: Every token simultaneously encodes local details and a global context.
ii. Versatility: The method herein applies universally to temporal signals (ECGs) and spatial data (e.g., CT scans, ultrasounds).
iii. AI-Friendly Structure: Tokens are optimally formatted for transformer-based models, reducing the need for extensive preprocessing.

Time series data is at the core of many scientific and industrial applications, from financial forecasting to medical diagnostics. The ability to effectively tokenize and represent this data determines how well AI models can interpret and analyze patterns over time. However, conventional tokenization approaches-which typically segment time series data into discrete parts-often struggle with preserving the full complexity of signals that exhibit wave-like behavior, such as electrocardiograms (ECG), ultrasound, and other physiological waveforms. In contrast, the Quantum Wave Function Tokenization scheme of the present disclosure (e.g., executed by the Q-PSI tokenizer 220 discussed above) treats the data as continuous wave functions. In more detail, instead of dividing time series into fixed segments, Q-PSI models the underlying waveform structure by fitting wave-like components, sometimes using Gaussian functions, to preserve the shape, morphology, and frequency characteristics of the original signal.

Importantly, this approach captures and preserves both the time-domain and frequency-domain characteristics of the signal, making it particularly well-suited for biomedical applications and offers more interpretability and depth of information retained from the original signal. In that regard, medical applications such as ECG, EEG, ultrasound, and other bio-signals contain oscillatory behavior. Traditional tokenization methods often struggle to capture subtle waveform variations, such as minor changes in ST-segment elevation in ECGs or Doppler shifts in ultrasound that indicate pathological conditions. Here, by treating the signal as a continuous function, Q-PSI retains the fundamental properties of waves, including phase relationships, frequency modulation, and waveform morphology. This makes it especially valuable in fields where signal continuity and frequency composition are essential to diagnosis. Q-PSI also aligns well with human interpretation of biomedical signals. Clinicians do not merely look at individual segments of an ECG or ultrasound waveform. Instead, they analyze the holistic structure of the waveforms, including their shapes, oscillations, amplitudes, and frequency changes over time. By providing a tokenization framework that mirrors this human understanding, Q-PSI makes AI-generated insights more interpretable and clinically useful.

While ECGs are a natural application of Quantum Wave Tokenization, other types of medical applications such as ultrasound imaging and EEG analysis also benefit from this approach. In ultrasound, both Doppler shifts and continuous wave characteristics provide valuable diagnostic insights. Standard tokenization methods used in AI-assisted ultrasound interpretation often fail to capture these shifts, leading to AI models that miss subtle vascular abnormalities or fetal cardiac issues. By encoding frequency changes and waveform oscillations in a physics-informed manner, Q-PSI enables transformer models to better understand continuous flow dynamics in Doppler ultrasound, leading to more accurate AI-driven diagnostics. Similarly, in EEG analysis, traditional segmentation-based methods tend to overlook phase coupling and oscillatory synchrony between different brain regions, which are critical markers in neurological disorders. Q-PSI, by treating EEG signals as coherent wave functions, allows models to better capture brainwave phase interactions and spectral changes over time, thereby leading to better seizure prediction and cognitive state analysis.

Referring now to Fig. 5, a process flow 500 for converting raw medical data into tokens via quantum waves is illustrated. In other words, the process flow 500 may be performed by the Q-PSI tokenizer 220 discussed above. The Q-PSI tokenizer 220 may be implemented using a combination of software code and hardware module(s), for example, specialized computer software code executing on one or more central processing units (CPUs) and/or one or more graphics processing units (GPUs). The process flow 500 addresses the challenge of extracting meaningful features from complex biosignals (like ECG traces) or other raw data streams, because before any high-level reasoning (e.g., performed by the M1 LLM and/or the M2 LLM discussed above) occurs, the input data needs to be converted into a structured representation.

In a step 510 of the process flow 500, raw medical data is collected and preprocessed. For example, the raw medical data may include the raw patient data 310 of Fig. 3, which may be collected or generated by a medical device such as the medical device 210 of Figs. 2-3 and/or the wireless communication device 110 of Fig. 1. In some embodiments, the raw medical data may include ECG data, EEG data, MRI data, RF signal data, X-ray data, CT/CAT scan data, ultrasound data, etc. The raw medical data may also come from multiple channels or leads and/or may be multi-modal (e.g., including more than one type of medical data). In some embodiments, the preprocessing of the step 510 may include filtering, baseline detection, noise reduction, ROI (region of interest) detection, segmentation, or a combination of them. For example, noise or signal distortions may be removed from the raw medical data, and/or the peaks and/or baselines of the signals in the raw medical may be identified as a part of the preprocessing of the step 510.

It is understood, however, that the process flow 500 (e.g., for generating quantum wave tokens) is not limited to raw medical data. In other embodiments, the step 510 may be performed to access or obtain non-medical data, such as textual data, semantic data, and/or other natural-language inputs. As non-limiting examples, these textual/semantic/natural-language inputs may include: physician notes, medical descriptions, psychological assessments, clinical summaries, radiology reports, psychological or psychiatric evaluations, patient self reports, or any other narrative or descriptive medical content. These other types of data are also valid input modalities for the Q-PSI tokenizer of the present disclosure, with LLM-based semantic quantization described as a permitted mechanism for generating the wave-function parameters. In certain embodiments, the input data associated with step 510 comprises unstructured natural-language text, such as physician notes, narrative clinical assessments, psychological evaluations, or diagnostic descriptions, which are processed by a language-model-based component of the quantum wave tokenizer to produce vectorized representations suitable for wave-function fitting, where the vectorized representations and the wave-function fitting will be discussed in more detail below.

In a step 530 of the process flow 500, the preprocessed raw medical data obtained as a result of the performance of the step 510 undergoes a vectorization process. As a part of the vectorization, continuous or discrete data points (e.g., of the preprocessed raw medical data) are transformed into an ordered, numerical array or vector. Fig. 5 illustrates a simplified example of a three-dimensional vector space corresponding to a cube defined by an X-axis, a Y-axis, and a Z-axis, where the X-axis, Y-axis, and the Z-axis are orthogonal to one another. The vectorized signals, such as vectorized signals 540 and 541, are plotted as three-dimensional vectors in such a vector space. However, it is understood that the vector space may comprise more than three dimensions, and thus the vectorized signals may also be more than three-dimensional.

In an embodiment, the preprocessed raw medical data comprises ECG data, and a continuous ECG signal may be segmented into RR intervals using R-peak detection. Each segment is then vectorized to obtain a two-dimensional (2D) angle-magnitude representation by fitting an inverse solution for a patient-specific conversion matrix (e.g., from leads to vector angles). In another embodiment, the preprocessed raw medical data comprises ultrasound data, and each 2D frame from an ultrasound probe is parsed for regions of interest (ROI) in a radial coordinate system to encode spiral token center sequence and angle. Fitting of 2D Gaussians detects edges and intensities. In yet another embodiments, the preprocessed raw medical data comprises MRI data, and 3D volumetric data of the MRI data is sliced or handled in full 3D, with pre-segmentation algorithms highlighting relevant anatomical structures around a central point.

In some embodiments, the vectorization of step 530 is performed by a transformer-based large language model (LLM) which transforms the textual input into numerical semantic features. These features may include latent embeddings, semantic coordinate vectors, or explicitly extracted semantic attributes representing the meaning of the text.

In a step 550 of the process flow 500, the vectorized signals obtained as a result of the performance of the step 530 undergo a quantum wave fitting, where a quantum wave has a plurality of parameters that include (but may not be limited to) amplitude, time, and angle. As such, the quantum wave is at least three-dimensional. In order to represent the three-dimensional information of the quantum wave in a two-dimensional quantum wave plots 560 of Fig. 5, the angle parameter may be assigned to different colors (illustrated as different types of shades in Fig. 5), while the amplitude parameter corresponds to the amplitude (i.e., Y-axis) of the quantum wave plots 560, and the time parameter corresponds to the time (i.e., X-axis) of the quantum wave plots 560. For example, if the vectors have the same amplitude and time information but the angle information would change, then the resulting quantum wave plots 560 would not change its overall shape, but the color (e.g., shade) of the quantum wave plots 560 would vary.

The name "quantum wave" comes from the approach of fitting segments of the input signal (e.g., the vectorized signals) with quantum-inspired wavelets or basis functions. For example, the Q-PSI tokenizer 220 discussed above decomposes an ECG signal into a series of component waves (akin to modeling the signal as a combination of known wave shapes, such as Gaussians or Hermite functions). By doing so, it captures physiologically relevant characteristics (amplitude, phase, frequency content of each wave component, etc.) that are not apparent from raw pixel data. A subsequent Feature Extractor then derives higher-level features from these components - such as durations of intervals (PR interval, QRS duration), amplitudes of peaks, heart rate variability measures, and morphological descriptors like axis or waveform shape. The end result is a rich vector of features that concisely represents the patient's ECG in terms of clinically relevant metrics.

In some embodiments, the tokenizer uses Gaussian or other pA-RAMetric (e.g., parametric) wave functions to model features in the data. Key parameters can include, but are not limited to:
- Amplitude or Peak Magnitude: Reflects signal intensity (ECG wave amplitude, grayscale intensity for ultrasound, etc.).
- Phase Angle: May correspond to 3D vector angle in ECG or probe angle and location in radial coordinates in ultrasound.
- Width or Spread: Defines the duration or spatial extent of a structure. In ECG, this might be the temporal width of the T wave; in ultrasound, the spatial thickness of an organ boundary.
- Location: The time offset within the RR interval or the distance from center coordinates in 2D/3D imaging.

The Quantum Wave Function Tokenizer (QWFT) (also interchangeably referred to as Q-PSI above and below) algorithm herein iteratively optimizes each wave function parameter (e.g., via gradient descent or least squares methods) to minimize the error between the fitted wave function and the actual signal or image data. Wave function fitting is the process of adjusting a mathematical form (such as a linear combination of basis functions) so that the resulting wave function best represents a quantum system. By way of a non-limiting example, this can involve basis set expansion (e.g., using predefined basis functions such as Gaussian-type orbitals to construct approximate wave functions); least-squares fitting to match a trial wave function to a known or target wave function (e.g., from experimental data or a higher-level theory); and/or fitting to potential energy surfaces (e.g., PES or optimizing the wave function so it gives energy values that closely match known PES data).

Parametric Optimization involves optimizing the parameters within the wave function (or within the Hamiltonian/operator models) to minimize some cost function - often the energy of the system. Methods for doing so include, but are not limited to: (a) the Variational Principle (used in quantum mechanics to find the ground state by minimizing the expectation value of the Hamiltonian); (b) Gradient-Based Optimization (e.g., Newton-Raphson, conjugate gradient, or quasi-Newton methods for optimizing orbital parameters); (c) Stochastic Methods (e.g., genetic algorithms or simulated annealing, used for global optimization in complex systems), and (d) Machine Learning Approaches (e.g., neural network wave functions such as FermiNet or PauliNet) using deep learning to optimize parameters in a data-driven way.

In this manner, the time-dependent precession of the vectors is modeled as a set of wave functions representing the heart's electrical activity as a sum of quantum waves. These wave functions form structural complexes that naturally correspond to known ECG features such as P-waves, QRS complexes, and T-waves. Each wave function is defined by precise parameters-angle, amplitude, time, and period-providing transformers with an intuitive way to interpret the data. This approach not only enables the detection of specific features, but also facilitates their quantification, such as measuring the QRS complex width or the spatial vector shape of a T-wave across multiple heartbeats, with exceptional clarity.

It is understood that the wave-function fitting of step 550 can also operate on semantic features derived from LLMs. This allows an LLM prompt to function as a "semantic wave-parameter estimator." In certain embodiments, the system herein fits parametric wave functions not only to quantitative signals but also to vectorized semantic features derived from textual input by the LLM. For example, the system may treat semantic intensity, polarity, context-orientation, or meaning-derived attributes as amplitude, phase, angle, width, or location parameters of the fitted wave functions. Moreover, in certain embodiments, the fitting of the parametric wave functions may be performed by a language model executing a structured prompting procedure, which outputs numerical parameter values such as amplitude, phase, angle, width, or location derived from semantic content of the text. Such parameters may be used directly as wave-function coefficients.

The advantage of the quantum wave fitting approach of the present disclosure is at least twofold: lossless compression and semantic clarity. Unlike a generic neural network ingesting raw signals (which might learn intermediate features but in an opaque way), the output features herein are explicit and meaningful to human experts. For example, one feature might be "P-wave amplitude in lead II" or "QT interval duration" - things a cardiologist directly understands. This not only aids interpretability but also provides a structured input for the downstream Random Forest and LLM components. By converting the raw data into a structured form, the present disclosure significantly reduces the input complexity (from thousands of time-series points to a few hundred key features) while preserving the critical information needed for diagnosis. In essence, the Q-PSI tokenizer 220 acts as the sensory preprocessing of the overall system of the present disclosure, analogous to how the human eye's retina preprocesses visual signals or the cochlea preprocesses sound. It ensures that the subsequent reasoning modules (e.g., the M1 and M2 LLMs) are dealing with a vector of features that directly correlate with diagnostic concepts.

In some embodiments, Q-PSI tokenizer is domain-specific (e.g., the specific wave-fitting approach is tailored to ECG in this case). However, the principle can generalize: for any domain with complex raw data (e.g. chemical spectra, sensor readings, genome sequences), a specialized tokenizer can be designed to extract structured features relevant to that domain. The modular architecture of the present disclosure allows swapping in different front-end tokenizers. The takeaway is that the system of the present disclosure does not treat raw data as an undifferentiated blob - it imposes a meaningful structure upfront, which is crucial for the system's determinism and transparency. By the time the data passes through the Q-PSI tokenizer 220, the rest of the system can reason in terms of "clinical parameters" rather than uninterpretable signal values. This is a stark contrast to end-to-end deep learning, and it addresses one cause of black-box behavior by making the input representation itself explainable.

In a step 570 of the process flow 500, the quantum waves obtained from step 550 undergo a clustering and tokenization process, in which discrete tokens are created to represent clinically significant features. In more detail, upon fitting, each wave function's parameters become tokens-structured data that can be used as input to LLMs or other AI models. For instance, in one embodiment, an ECG Token may be represented as follows:

```
      {
      Wave Region: 'T-wave', amplitude:
       0.8mV,
      phaseAngle: 45°/111°, // from vector ECG
      width: 80ms,
      timeOffset: 320ms from R-peak
      }
```

In another embodiment, an ultrasound Token may be represented as follows:

```
            {
             structureType: 'fetal head boundary',
             amplitude: 120 (greyscale),
             phaseAngle: 90°, probe angle 30°
             width: 15 pixels,
             location: (d=120)
            }
```

Regardless of whether the tokens are ECG tokens or ultrasound tokens (or other types of tokens corresponding to different medical technologies), they are both human-readable and reflect clinically meaningful attributes. With respect to variability analysis and classification, the system of the present disclosure can compare token sets across multiple time points or frames:
∘ **ECG:** By comparing each RR interval's wave tokens to an average or reference set, abnormal morphologies (e.g., arrhythmic beats, ST changes) can be detected. This comparison is particularly valuable for long-term trends such as T-wave alternans, which may signal lethal arrhythmias.
∘ **Ultrasound:** Comparing wave tokens across a sequence of frames with different scanning angles helps track fetal organ growth or movement in real time.
∘ **MRI:** Tracking volumetric wave tokens over multiple slices can highlight lesions or morphological changes between scans.

The token outputs herein are also adapted for easy integration with transformative AI. For example, the QWFT outputs are designed for direct ingestion by advanced AI pipelines, particularly large language models. For instance, in the dual LLM system discussed above, these wave tokens can be continuously fed into the model's context window, enabling real-time interpretation alongside other sensor or textual data.

It is understood that the resulting quantum wave tokens associated with step 570 may represent the semantic meaning of the text. In some embodiments, the quantum wave tokens generated from textual input represent semantic quantum wave parameters reflecting clinical meaning, such as severity, stability, symptom intensity, diagnostic category, or other semantic features extracted by the LLM.

Note that the various use-cases of the text-based wave tokenization applies to clinical workflows, such as:
- labeling patient state ("mild," "moderate," "severe");
- extracting semantic descriptors;
- integrating narrative descriptions into diagnostic models;
- harmonizing textual and quantitative modalities for case-placement in the memory lattice.

In some embodiments, the textual quantum wave tokens are used to classify patient state, disease severity, psychological profile, or other medically relevant semantic content, allowing integration of narrative medical information with quantitative physiological signals in unified downstream machine-learning models.

It is also understood that the system discussed herein can ingest both quantitative physiological signals and unstructured text. The system can tokenize them through the same wave-function-parameter framework, so that all tokens may populate the downstream classifier or graph-memory system. In some embodiments, the quantum wave tokenizer herein may be multimodal, receiving both quantitative time-series signals (e.g., ECG) and qualitative natural-language medical descriptions. Both modalities are converted into wave-parameterized quantum tokens that can be uniformly processed by downstream classifiers, random forest models, transformers, or graph-based memory architectures.

As discussed above, traditional medical data processing-even techniques that utilize machine learning models like convolutional neural networks to handle raw pixel data or raw time-series signals-may still struggle with:
1. High Dimensionality: Large volumes of data (e.g., continuous ECG, dense ultrasound frames, 3D MRI volumes) can exceed the parallel input limit of many machine learning models.
2. Contextual Interpretation: Existing systems often require complex feature engineering or manual labeling to interpret subtle but clinically significant variations (e.g., minor changes in T-wave morphology on ECG, faint structures in ultrasound, or small lesions on MRI).
3. Integration with Medical Expertise: Conventional models can underutilize domain knowledge from cardiology, radiology, or obstetrics, resulting in interpretative gaps that limit diagnostic potential.

As such, bridging the gap between raw medical data and an LLM-friendly representation had been a key challenge. The Quantum Wave Function Tokenizer (QWFT) scheme discussed above addresses the previous challenges by converting medical signals and images into parameterized wave function tokens that align with defined clinical features (e.g., P waves, T waves in ECG; structural shapes in ultrasound; volumetric features in MRI). As discussed above in association with Fig. 5, the QWFT scheme herein transforms complex medical data, signals or images into a compact, clinically aligned representation. By modeling portions of data, signals, or images as parameterized wave functions, the QWFT herein can be configured to:
1. **Reduce Data Dimensionality:** Instead of raw data, each relevant feature (e.g., a detected ECG wave, an anatomical boundary on ultrasound, or a volumetric contour in MRI) is represented by a set of wave function parameters-amplitude, width, phase angle, location, etc. In the case of the ECG, the phase angle represents the dominating vector direction of the best fitted voltage vector defined by all leads and through an averaged RR-interval of the recording, and the time represents the recording time, while the token sequence is defined by their temporal sequence. For the imaging applications the token angle is the position angle of the quantum wave center on a radial coordinate system around a central point, and the sequence of tokens is a spiral track from this point, while all tokens of one image have the same time.
2. **Enhance Interpretability:** These quantized parameters sets are defined to map naturally to known clinical concepts (e.g., intervals and waves in ECG, structural dimensions in ultrasound, lesion boundaries in MRI), making the output tokens intrinsically meaningful to both human clinicians and knowledge-based AI models.
3. **Facilitate Rapid, Real-Time or Near-Real Time Processing:** By using efficient fitting, the QWFT can update its tokenized representation in near real-time, making it suitable for continuous monitoring applications such as fetal ultrasound imaging or real-time arrhythmia detection.
4. **Integrate Seamlessly with Advanced AI:** The output tokens can be fed into a large language model or a transformer-based system (such as the dual LLM system discussed above) to leverage multimodal and contextual awareness. This allows the combined system to reason about both the wave function tokens and existing clinical knowledge simultaneously.
5. **Cover Extended ECG Analysis:** Long-duration ECG monitoring becomes more actionable because subtle morphological changes-like T-wave heterogeneities predictive of ventricular arrhythmias or P-wave variations indicative of atrial fibrillation recurrence-are captured in the QWFT parameters, significantly improving diagnostic accuracy and risk prediction.

Note that the QWFT disclosed and described herein may be employed in conjunction with one, two or more LLMs, and one or more retrieval-augmented generators (RAGs), where the QWFT receives ECG data or signals, tokenizes such data or signals, and then provides as outputs therefrom tokens to the one, two or more LLMs, which may be operably connected to the one or more RAGs. In some embodiments, the tokenized output from the Q-PSI tokenizer (such as the result from the step 570 of the process flow 500) may be used by the dual LLMs of the present disclosure to perform diagnostics. For example, in one embodiment, the M1 LLM receives each tokenized output as 1.7-second, tokenized ECG chunk, quickly identifying features such as ectopic beats, QRS morphology, or P-wave supraventricular premature beats. The M1 LLM then produces raw inferences and compact feature descriptors that are passed to the M2 LLM, which compiles these data into a broader timeline spanning tens of minutes.

In one embodiment, the M2 LLM continuously updates its context with incoming M1 LLM descriptors, examining subtle, slow-forming events like periodic clusters of atrial contractions or prolonged PR intervals. This "long-term" analysis reveals emerging conduction changes that may not be discernible from a single 1.7-second snapshot. Once a recording is complete, the M2 LLM finalizes an overall statistical summary of the session. This summary and all associated patient data are then appended to a long-term memory (LTM) repository so that the entire episode, along with the patient's history, can be retrieved in subsequent sessions.

In one embodiment, finetuning the M1 LLM and the M2 LLM relies on labeled ECG datasets, typically snippets of 1.7 seconds each, drawn from patients who did or did not experience atrial fibrillation (AF) recurrence post pulmonary vein isolation (PVI). The M1 LLM is first exposed to large unlabeled ECG corpora to learn general features. The M1 LLM is then trained on the labeled snippets for specific arrhythmia indicators. The M2 LLM is trained to integrate the M1 LLM's short-term embeddings over extended durations, enabling recognition of how transient events accumulate into higher-risk patterns over minutes. By dividing these responsibilities, the M1 LLM excels in detecting second-to-second nuances, while the M2 LLM becomes adept at correlating them with longer-term outcomes.

In one embodiment, the pseudocode of a QWFT pipeline for ECG data is listed below:

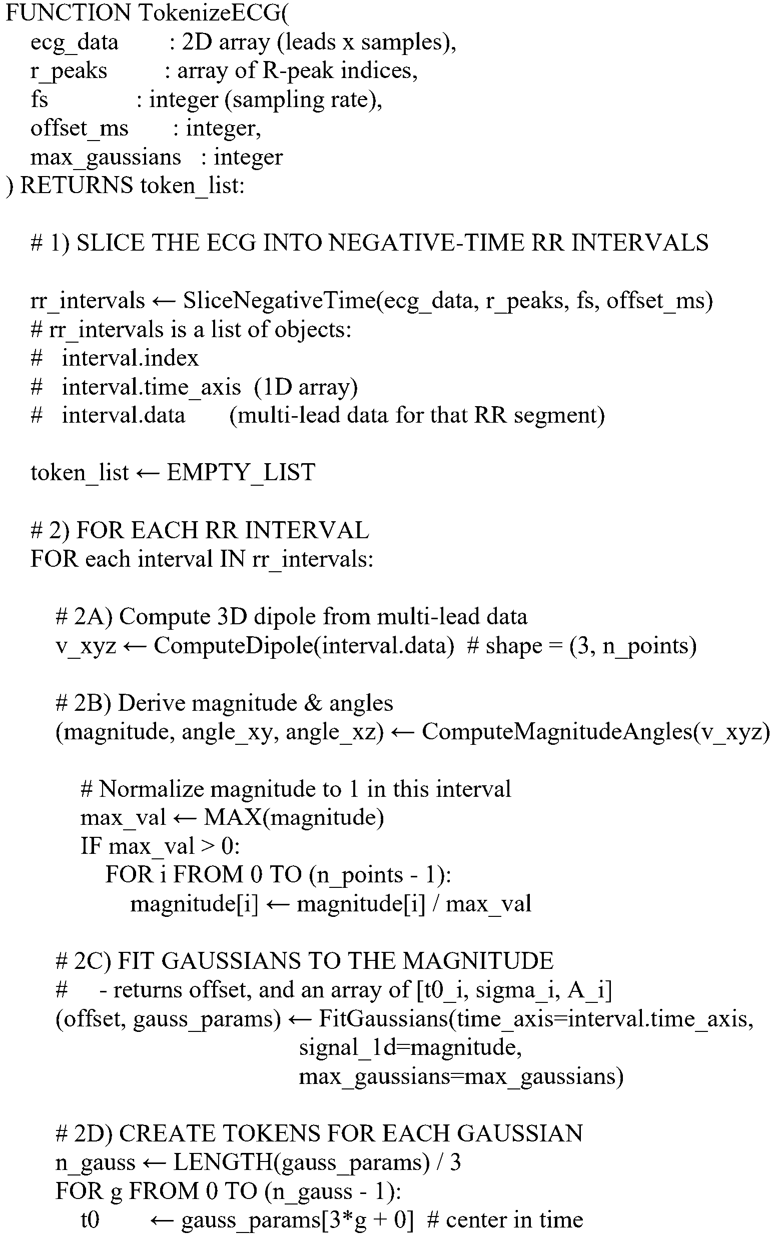

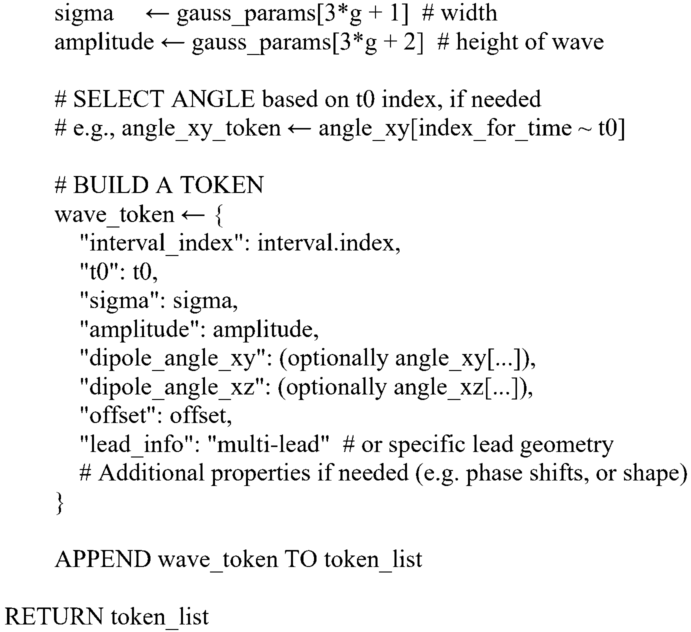

In some embodiments, the QWFT/Q-PSI scheme of the present disclosure is implemented with hardware acceleration. For example, an analog ASIC or specialized GPU can accelerate the fitting of wave functions (e.g., step 550 of Fig. 5) to high-dimensional data (e.g., 2D ultrasound frames, 3D MRI slices). In addition, parallelized fitting algorithms enable near-instantaneous updates, allowing the QWFT to handle fast physiological signals or real-time imaging. As such, the QWFT/Q-PSI scheme herein may be particularly useful in the field of cardiology (Holter monitor real-time analysis, stress ECG interpretation, prediction of sudden cardiac death, or telemedicine for arrhythmia detection), obstetrics (monitoring fetal growth via ultrasound in near real-time, comparing tokenized wave structures against typical developmental norms), and/or radiology (automated segmentation and anomaly detection in MRI, with advanced AI cross-referencing prior scans and patient records).

Discussed below are some concrete examples of the implementation of the QWFT/Q-PSI scheme herein, which may involve the performance of the steps 510-570 of Fig. 5 discussed above. In a first example, the QWFT/Q-PSI scheme is implemented for an ECG Tokenizer for Arrhythmia Detection and Risk Prediction. First, 24-hour Holter ECG data is collected at 500 Hz. R-peaks are identified, and RR intervals are segmented out. An average RR interval is computed to establish reference wave function parameters for P-wave, QRS complex, and T-wave. For each new RR interval, the QWFT fits wave functions, compares them to the reference, and flags significant deviations. In terms of risk stratification with respect to T-Wave Heterogeneity: the system herein detects subtle changes in T-wave amplitude, width, or phase angle. These morphological fluctuations (e.g., T-wave alternans) can signal higher risk for ventricular arrhythmias or sudden cardiac death. In terms of risk stratification with respect to P-Wave Heterogeneity: the system herein analyzes P-wave shape and timing across thousands of beats. Small irregularities or rare premature beats can predict the likelihood of atrial fibrillation recurrence, especially after pulmonary vein isolation procedures. A downstream LLM processes these token sequences over time (e.g., 24 hours of continuous data) and outputs a risk assessment, highlighting patients who may benefit from additional interventions, such as electrophysiological mapping or medication adjustments.

In a second example, the QWFT/Q-PSI scheme is implemented for an Ultrasound-Based Fetal Assessment. First, a 2D ultrasound probe captures cross-sectional images of a fetus. The QWFT then fits 2D Gaussian wave functions to represent edges of the skull, abdomen, and limbs. The probe angle is recorded as phase angle for each fitted wave function, providing orientation context. The resulting tokens are fed into a real-time LLM system that cross-references standard fetal growth charts. If the head circumference tokens deviate from the norm, the system raises an alert.

In a third example, the QWFT/Q-PSI scheme is implemented for MRI Lesion Tracking. First, a 3D MRI scan of the brain is obtained. Automated segmentation then highlights a suspicious lesion. The QWFT fits a 3D wave function (e.g., Gaussian ellipsoid) to approximate the lesion's shape and volume. Tokens describing the lesion parameters (size, location, approximate shape) are fed into a transformer that compares current and historical scans, assessing growth rates. Clinicians receive an automated summary referencing known lesion progression patterns.

Some of the advantages of the QWFT/Q-PSI scheme over the prior art may include (but is not limited to):
- Clinically Aligned Representation: Unlike raw data vectors or pixel-based approaches, wave tokens map directly to clinically recognized features, facilitating immediate interpretability.
- Reduced Complexity: Lower data dimensionality means more efficient processing, especially crucial for real-time applications.
- Adaptable Across Modalities: The same tokenization concept works for temporal signals (ECG) and spatial/volumetric data (ultrasound, MRI).
- Seamless Integration with LLMs: Because tokens are structured and annotated with clinically meaningful parameters, large language models can interpret them with minimal additional feature engineering.
- Improved Predictive Power: Long-duration ECG analysis becomes more actionable for high-risk event prediction (e.g., sudden cardiac death, atrial fibrillation recurrence) through robust wave-based feature extraction.

Various concepts of the present disclosure may be applied in a medical context, as well as non-medical contexts. In a non-limiting medical context example, the present disclosure may be applied to perform medical diagnostics with episodic recall. In more detail, an ECG Data Stream may be accessed. A quantum wave function tokenizer provides the M1 LLM with tokens describing each RR interval in real time. The M1 LLM performs immediate pattern recognition (e.g., checking T-wave heterogeneity). As long-term memory, the M2 LLM retrieves prior episodes of the patient's ECG (e.g., last month's recordings) upon seeing keywords like "arrhythmia." The M2 LLM also references an expert database (e.g., standard guidelines for diagnosing atrial fibrillation recurrence). In terms of episodic storage, when the session ends or a major change occurs (e.g., new medication started), M1 LLM's context is condensed into a summary and stored by the M2 LLM for future recall.

In a robotic navigation example, sensors such as lidar, cameras, or speed/inertia detectors are used to obtain initial data. The initial data is read by the M1 LLM as tokens. The M1 LLM generates motor commands on the fly, guiding the robot around obstacles. If the M1 LLM identifies an unfamiliar obstacle type, the M2 LLM triggers an internal knowledge base query (e.g., "obstacle classification: partial occlusion scenario"). The M2 LLM references stored "episodes" of prior near-collision situations, merges the solution path into M1 LLM's context, and the robot navigates safely.

In an industrial monitoring system example, real-time data feeds are obtained (e.g., from an assembly line), and the M1 LLM processes sensor tokens (temperature, pressure, vibration) from the assembly line. The M2 LLM detects unusual vibration patterns and queries an expert database on mechanical fault thresholds, returning relevant guidelines to the M1 LLM. As episodic summaries, the M2 LLM stores M1 LLM's summary after each significant maintenance or system alert, building an episodic knowledge base for future anomaly detection.

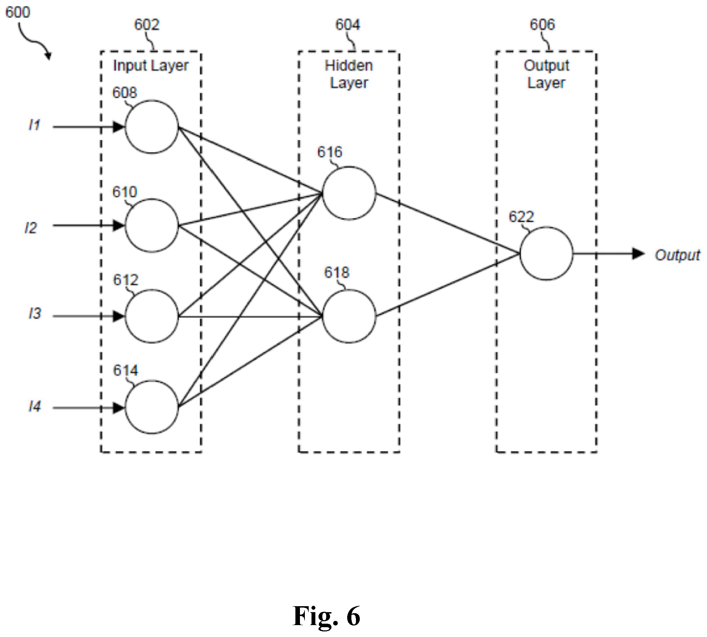

As discussed above, machine learning may be used to implement various components of the present disclosure. In some embodiments, the LLMs may be implemented at least in part via an artificial neural network. In that regard, Fig. 6 illustrates a simplified example artificial neural network 600. As shown, the artificial neural network 600 includes three layers - an input layer 602, a hidden layer 604, and an output layer 606. Each of the layers 602, 604, and 606 may include one or more nodes. For example, the input layer 602 includes nodes 608-614, the hidden layer 604 includes nodes 616-618, and the output layer 606 includes a node 622. In this example, each node in a layer is connected to every node in an adjacent layer. For example, the node 608 in the input layer 602 is connected to both of the nodes 616-618 in the hidden layer 604. Similarly, the node 616 in the hidden layer is connected to all of the nodes 608-614 in the input layer 602 and the node 622 in the output layer 606. Although only one hidden layer is shown for the artificial neural network 600, it has been contemplated that the artificial neural network 600 used to implement a part of the M1 LLM or the M2 LLM, and the M1 LLM or the M2 LLM may include as many hidden layers as necessary.

In this example, the artificial neural network 600 receives a set of input values and produces an output value. Each node in the input layer 602 may correspond to a distinct input value. For example, when the artificial neural network 600 is used to implement a machine learning module, each node in the input layer 602 may correspond to a distinct attribute of an ECG signal (or other types of incoming signals).

In some embodiments, each of the nodes 616-618 in the hidden layer 604 generates a representation, which may include a mathematical computation (or algorithm) that produces a value based on the input values received from the nodes 608-614. The mathematical computation may include assigning different weights to each of the data values received from the nodes 608-614. The nodes 616 and 618 may include different algorithms and/or different weights assigned to the data variables from the nodes 608-614 such that each of the nodes 616-618 may produce a different value based on the same input values received from the nodes 608-614. In some embodiments, the weights that are initially assigned to the features (or input values) for each of the nodes 616-618 may be randomly generated (e.g., using a computer randomizer). The values generated by the nodes 616 and 618 may be used by the node 622 in the output layer 606 to produce an output value for the artificial neural network 600. When the artificial neural network 600 is used to implement the machine learning module, the output value produced by the artificial neural network 600 may indicate a likelihood of an event.

The artificial neural network 600 may be trained by using training data. For example, the training data herein may include ECG data collected from previous patients and the observed outcomes and/or conditions of the previous patients. By providing training data to the artificial neural network 600, the nodes 616-618 in the hidden layer 604 may be trained (adjusted) such that an optimal output (e.g., determining a value for a threshold) is produced in the output layer 606 based on the training data. By continuously providing different sets of training data, and penalizing the artificial neural network 600 when the output of the artificial neural network 600 is incorrect, the artificial neural network 600 (and specifically, the representations of the nodes in the hidden layer 604) may be trained (adjusted) to improve its performance in data classification. Adjusting the artificial neural network 600 may include adjusting the weights associated with each node in the hidden layer 604. Other types of machine learning algorithms are not discussed in detail herein for reasons of simplicity.

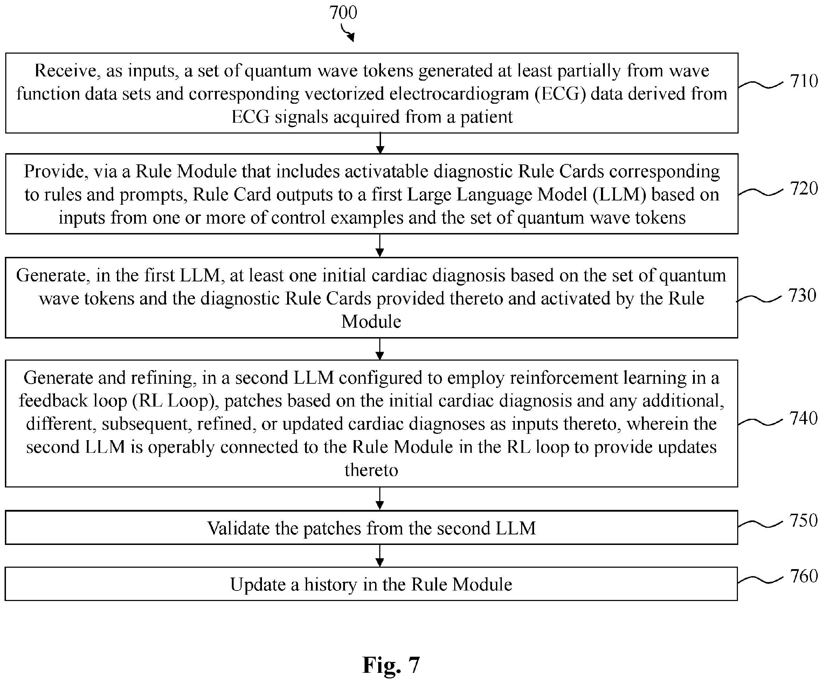

Fig. 7 is a flowchart illustrating a method 700 of generating a medically safe diagnosis via the dual LLM architecture discussed above. In some embodiments, one or more steps of the method 700 may be performed by, or correspond to, various components discussed above with reference to Figs. 1-4.

The method 700 includes a step 710 to receive, as inputs, a set of quantum wave tokens generated at least partially from wave function data sets and corresponding vectorized electrocardiogram (ECG) data derived from ECG signals acquired from a patient.

The method 700 includes a step 720 to provide, via a Rule Module that includes activatable diagnostic Rule Cards corresponding to rules and prompts, Rule Card outputs to a first Large Language Model (LLM) based on inputs from one or more of control examples and the set of quantum wave tokens. In some embodiments, the Rule Module and/or the Rule Cards may include, or correspond to, the skills, skill engine, and/or the skill universe discussed above. In some embodiments, the first LLM may include, or correspond to, the M1 LLM discussed above. In some embodiments, the Rule Module operates as at least one of: a Retrieval-Augmented Generation or Reinforcement Learning-Based Generator. In some embodiments, the diagnostic Rule Cards are editable.

The method 700 includes a step 730 to generate, in the first LLM, at least one initial cardiac diagnosis based on the set of quantum wave tokens and the diagnostic Rule Cards provided thereto and activated by the Rule Module. In some embodiments, the diagnostic Rule Cards comprise one or more first Rule Cards that comprise nearest neighbor personalized range information corresponding to the patient, and the at least one initial cardiac diagnosis is generated by the first LLM based on the one or more first Rule Cards. In some embodiments, the diagnostic Rule Cards comprise one or more first Rule Cards that comprise personalized thresholds computed on the basis of a local control cohort, and the at least one initial cardiac diagnosis is generated by the first LLM based on the one or more first Rule Cards.

The method 700 includes a step 740 to generate and refine, in a second LLM configured to employ reinforcement learning in a feedback loop (RL Loop), patches based on the initial cardiac diagnosis and any additional, different, subsequent, refined, or updated cardiac diagnoses as inputs thereto, wherein the second LLM is operably connected to the Rule Module in the RL loop to provide updates thereto. In some embodiments, the second LLM may include, or correspond to, the M2 LLM discussed above. In some embodiments, the second LLM in the RL Loop is configured to receive one or more of patient data, Rule Card information, the at least one initial cardiac diagnosis, and a reference cardiac diagnosis of the patient. In some embodiments, the second LLM in the RL Loop is configured to output the patches. The patches are configured to modify the rules, thresholds for the rules, and the prompts.

The method 700 includes a step 750 to validate the patches from the second LLM.

The method 700 includes a step 760 to update a history in the Rule Module. In some embodiments, the steps 750 and/or 760 may correspond to the validation process of Fig. 1.

It is understood that additional method steps may be performed before, during, or after the steps 710-760 discussed above. For example, the method 700 may include a step of generating at least one medically safe cardiac diagnosis based on: the set of quantum wave tokens; and computations and results provided by interactions between the Rule Module, the first LLM, and the second LLM operating in the RL Loop. As another example, the method 700 may include a step of repeating the receiving, the providing, the generating the at least one initial cardiac diagnosis, the generating the patches, the validating, and the updating until at least one medically safe diagnosis is generated and matches a reference cardiac diagnosis or until a predetermined number of iterations have been completed. As yet another example, the method 700 may include a step of comparing the at least one initial cardiac diagnosis and any subsequent cardiac diagnoses to a reference cardiac diagnosis. As a further example, the method 700 may include a step of providing at least one medically safe cardiac diagnosis based on a determination that the at least one initial or subsequent cardiac diagnosis matches the reference cardiac diagnosis. As another example, the method 700 may include a step of generating, via a quantum wave function tokenizer, the set of quantum wave tokens. Other steps may be performed by the method 700 but are not specifically discussed herein for reasons of simplicity.

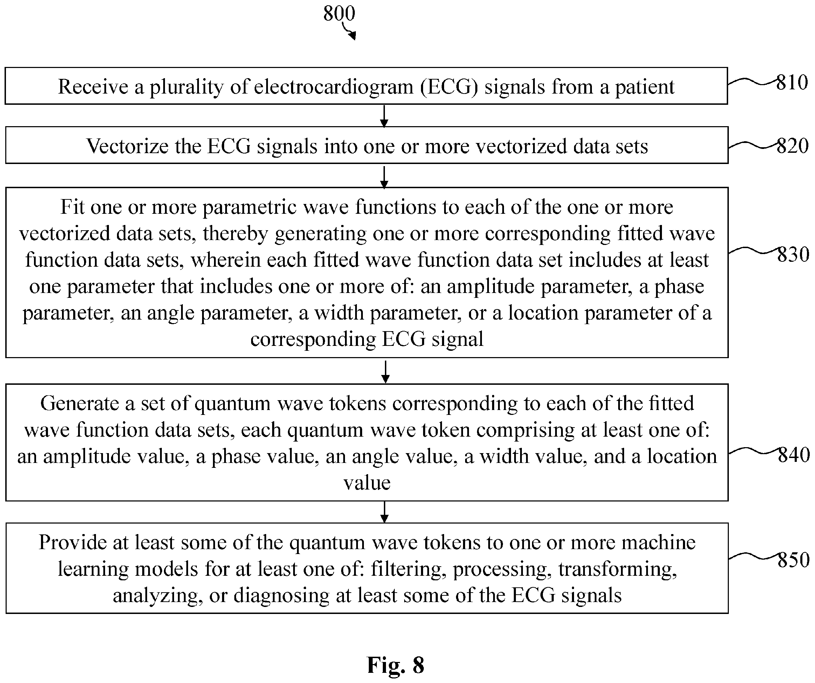

Fig. 8 is a flowchart illustrating a method 800 for generating a set of quantum wave tokens corresponding to a plurality of electrocardiogram (ECG) signals acquired from one or more locations on or in a patient. In some embodiments, one or more steps of the method 800 may be performed by, or correspond to, various components discussed above with reference to Figs. 1-5, such as the Q-PSI tokenizer 220.

The method 800 includes a step 810 to receive a plurality of electrocardiogram (ECG) signals from a patient. For example, the ECG signals may be received from the wireless communications device 110 of Fig. 1. In some embodiments, the wireless communications device 110 may include, or may be coupled to, a data acquisition module, a 12-lead ECG, a resting ECG system, a Holter ECG monitoring system, an ECG stress monitor, an ECG event monitoring system, or an ECG management system, which may be capable of extracting the ECG signals from the patient. In some embodiments, the step 810 may include the performance of the step 510 of Fig. 5.

The method 800 includes a step 820 to vectorize the ECG signals into one or more vectorized data sets. In some embodiments, the step 820 may include the performance of the step 530 of Fig. 5. In some embodiments, the vectorizing of step 820 is performed at least in part by applying a transformer-based large language model (LLM) to a natural-language text to generate semantic numerical representations.

The method 800 includes a step 830 to fit one or more parametric wave functions to each of the one or more vectorized data sets, thereby generating one or more corresponding fitted wave function data sets. Each fitted wave function data set includes at least one parameter that includes one or more of: an amplitude parameter, a phase parameter, an angle parameter, a width parameter, or a location parameter of a corresponding ECG signal. In some embodiments, the step 830 may include the performance of the step 550 of Fig. 5. In some embodiments, the wave fitting of step 830 is performed by a hardware-accelerating component, such as one more of analog ASICs, GPUs, or FPGAs.

In some embodiments, the parametric wave functions comprise one or more of: Gaussian-based parametric wave functions, Slater-Jastrow wave functions, neural network quantum state (NNQS) functions, or functions for minimizing energy expectation values. In some embodiments, the at least one parameter represents one or more of: T-wave alternans, T-wave variability, Q-wave segment duration, ST-segment elevation, prolonged QT-interval, or QRS-segment duration. In some embodiments, the parametric wave functions are fitted based on semantic attributes extracted from the natural-language text by the transformer-based LLM. In some embodiments, the location parameter represents a temporal or spatial extent of a portion of the ECG signals. In some other embodiments, the location parameter represents a temporal offset within one of the RR intervals. In some embodiments, the angle parameter corresponds to an angle of one of the ECG signals.

The method 800 includes a step 840 to generate a set of quantum wave tokens corresponding to each of the fitted wave function data sets, each quantum wave token comprising at least one of: an amplitude value, a phase value, an angle value, a width value, and a location value. In some embodiments, the step 840 may include the performance of the step 570 of Fig. 5. In some embodiments, the quantum wave tokens represent semantic meaning components of a medical narrative text, clinical narrative descriptions, medical opinions, or psychological evaluations.

In some embodiments, the one or more machine learning models comprises a first machine learning model and a second machine learning model. The first machine learning model is configured to receive the set of tokens generated from step 840. The first machine learning model comprises a transformer-based large language model configured to interpret the set of tokens alongside additional contextual data. The second machine learning model is configured to operate in conjunction with the first machine learning model.

The method 800 includes a step 850 to provide at least some of the quantum wave tokens to one or more machine learning models for at least one of: filtering, processing, transforming, analyzing, or diagnosing at least some of the ECG signals. In some embodiments, the one or more machine learning models generate, based on the quantum wave tokens, at least one cardiac diagnostic or analytical output regarding the patient. In some embodiments, a RAG (Retrieval-Augmented Generation) or a Reinforcement Generator (Reinforcement Learning-Based Generator) may be utilized in conjunction with the first machine learning model and/or the second machine learning model. In some embodiments, a convolutional neural network may be utilized in conjunction with at least one of the RAG, the first machine learning model, or the second machine learning model.

It is understood that additional method steps may be performed before, during, or after the steps 810-850 discussed above. For example, the method 800 may include steps of segmenting each ECG signal into RR intervals, which may be performed before the vectorizing of step 820. As another example, the method 800 may include a step of generating a two-dimensional angle-magnitude representation of a vectorized segment of the ECG signal by fitting an inverse solution for a patient-specific conversion matrix. In some embodiments, a real-time monitoring module is configured to update the token set for each new data frame or interval, thereby enabling continuous or near-continuous diagnostic monitoring or analysis.

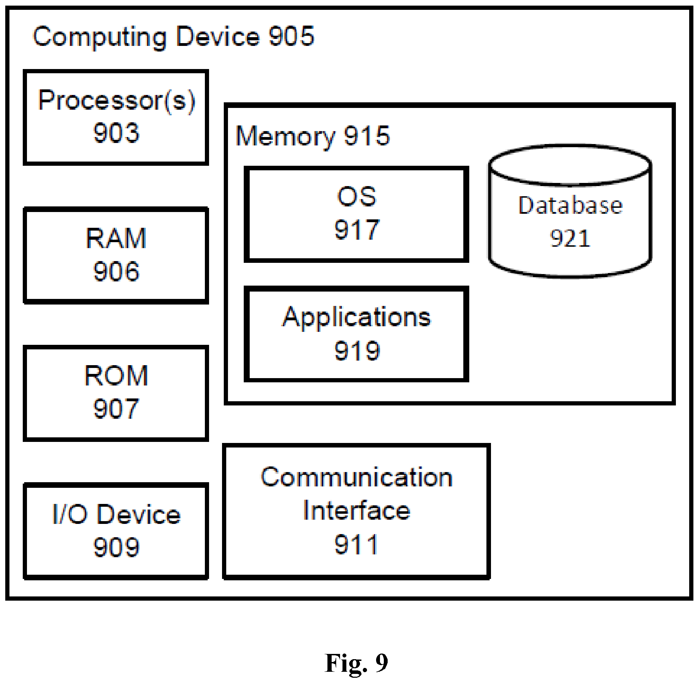

Turning now to Fig. 9, a computing device 905 that may be used with one or more of the computational systems is described. The computing device 905 may be used to implement various computing devices discussed above with reference to Figs. 1-8. For example, the computing device 905 may be used to implement the computer 120 of Fig. 1, the components of the framework 100 comprising the dual LLM architecture of Fig. 1, or various components of the process flows 200, 300, 400, and/or 500 of Figs. 2-5.

The computing device 905 may include one or more processors 903 for controlling overall operation of the computing device 905 and its associated components, including RAM 906, ROM 907, input/output device 909, communication interface 911, and/or memory 915. A data bus may interconnect processor(s) 903, RAM 906, ROM 907, memory 915, I/O device 909, and/or communication interface 911. In some embodiments, computing device 905 may represent, be incorporated in, and/or include various devices such as a desktop computer, a computer server, a mobile device, such as a laptop computer, a tablet computer, a smart phone, any other types of mobile computing devices, and the like, and/or any other type of data processing device.

Input/output (I/O) device 909 may include a microphone, keypad, touch screen, and/or stylus motion, gesture, through which a user of the computing device 905 may provide input, and may also include one or more speakers for providing audio output and a video display device for providing textual, audiovisual, and/or graphical output. Software may be stored within memory 915 to provide instructions to processor(s) 903 allowing computing device 905 to perform various actions. For example, memory 915 may store software used by the computing device 905, such as an operating system 917, application programs 919, and/or an associated internal database 921. The various hardware memory units in memory 915 may include volatile and nonvolatile, removable and non-removable media implemented in any method or technology for storage of information such as computer-readable instructions, data structures, program modules, or other data. Memory 915 may include one or more physical persistent memory devices and/or one or more non-persistent memory devices. Memory 915 may include, but is not limited to, random access memory (RAM) 906, read only memory (ROM) 907, electronically erasable programmable read only memory (EEPROM), flash memory or other memory technology, optical disk storage, magnetic cassettes, magnetic tape, magnetic disk storage or other magnetic storage devices, or any other medium that may be used to store the desired information and that may be accessed by processor(s) 903.

Communication interface 911 may include one or more transceivers, digital signal processors, and/or additional circuitry and software for communicating via any network, wired or wireless, using any protocol as described herein.

Processor(s) 903 may include a single central processing unit (CPU) in some embodiments, which may be a single-core or multi-core processor, or it may include multiple CPUs in other embodiments. In some embodiments, the processor(s) 903 may include one or more GPUs, in addition to, or in lieu of, the CPUs. The processor(s) 903 and associated components may allow the computing device 905 to execute a series of computer-readable instructions to perform some or all of the processes described herein. Although not shown in Fig. 9, various elements within memory 915 or other components in computing device 905, may include one or more caches, for example, CPU/GPU caches used by the processor 903, page caches used by the operating system 917, disk caches of a hard drive, and/or database caches used to cache content from database 921. For embodiments including a CPU/GPU cache, the CPU/GPU cache may be used by one or more processors 903 to reduce memory latency and access time. Processor(s) 903 may retrieve data from or write data to the CPU/GPU cache rather than reading/writing to memory 915, which may improve the speed of these operations. In some examples, a database cache may be created in which certain data from a database 921 is cached in a separate smaller database in a memory separate from the database, such as in RAM 906 or on a separate computing device. For instance, in a multi-tiered application, a database cache on an application server may reduce data retrieval and data manipulation time by not needing to communicate over a network with a back-end database server. These types of caches and others may be included in various embodiments, and may provide potential advantages in certain implementations of devices, systems, and methods described herein, such as faster response times and less dependence on network conditions when transmitting and receiving data.

Although various components of computing device 905 are described separately, functionality of the various components may be combined and/or performed by a single component and/or multiple computing devices in communication without departing from the invention.

It should be appreciated that like reference numerals are used to identify like elements illustrated in one or more of the figures, wherein these labeled figures are for purposes of illustrating embodiments of the present disclosure and not for purposes of limiting the same.

One aspect of the present disclosure involves a medical system. The medical system comprises: a plurality of computing devices, each of the computing devices including a non-transitory computer readable medium configured to store instructions executable by at least one processor associated therewith to receive as inputs thereto a set of quantum wave tokens generated at least partially from wave function data sets and corresponding vectorized electrocardiogram (ECG) data derived from ECG signals acquired from a patient, and to generate at least one initial cardiac diagnosis from the set of quantum wave tokens, generate and refine patches, based on the initial cardiac diagnosis and any subsequent cardiac diagnoses, validate the patches, and provide at least one medically safe cardiac diagnosis, the plurality of computing devices together being configured to perform actions that include: (a) receive the set of quantum wave tokens as inputs thereto; (b) from a Rule Module that includes diagnostic Rule Cards corresponding to rules and prompts, provide Rule Card outputs to a first Large Language Model (LLM) based on inputs from one or more of control examples and the set of quantum wave tokens; (c) in the first LLM, generate the at least one initial cardiac diagnosis from the set of quantum wave tokens and the diagnostic Rule Cards provided thereto and activated by the Rule Module; (d) in a second LLM configured to employ reinforcement learning in a feedback loop (RL Loop), generate and refine patches based on the initial cardiac diagnosis and any additional, different, subsequent, refined, or updated cardiac diagnoses as inputs thereto, wherein the second LLM is operably connected to the Rule Module in the RL loop to provide updates thereto; (e) validate patches from the second LLM; and (f) update at least one history in the Rule Module.

Another aspect of the present disclosure involves a method. The method includes: receiving, as inputs, a set of quantum wave tokens generated at least partially from wave function data sets and corresponding vectorized electrocardiogram (ECG) data derived from ECG signals acquired from a patient; providing, via a Rule Module that includes activatable diagnostic Rule Cards corresponding to rules and prompts, Rule Card outputs to a first Large Language Model (LLM) based on inputs from one or more of control examples and the set of quantum wave tokens; generating, in the first LLM, at least one initial cardiac diagnosis based on the set of quantum wave tokens and the diagnostic Rule Cards provided thereto and activated by the Rule Module; generating and refining, in a second LLM configured to employ reinforcement learning in a feedback loop (RL Loop), patches based on the initial cardiac diagnosis and any additional, different, subsequent, refined, or updated cardiac diagnoses as inputs thereto, wherein the second LLM is operably connected to the Rule Module in the RL loop to provide updates thereto; validating the patches from the second LLM; and updating a history in the Rule Module.

Yet another aspect of the present disclosure involves a method. The method includes: generating, via a quantum wave function tokenizer, a set of quantum wave tokens based on raw medical data obtained from a patient; providing, via a Rule Module that includes activatable diagnostic Rule Cards corresponding to rules and prompts, Rule Card outputs to a first Large Language Model (LLM) based on inputs from one or more of control examples and the set of quantum wave tokens; generating, in the first LLM, at least one initial cardiac diagnosis based on the set of quantum wave tokens and the diagnostic Rule Cards provided thereto and activated by the Rule Module; generating and refining, in a second LLM configured to employ reinforcement learning in a feedback loop (RL Loop), patches based on the initial cardiac diagnosis and one or more updated cardiac diagnoses, wherein the second LLM is operably connected to the Rule Module in the RL loop to provide updates thereto; validating the patches generated by the second LLM; and updating a history in the Rule Module.

Another aspect of the present disclosure involves a quantum wave tokenizer system configured to generate a set of quantum wave tokens corresponding to a plurality of electrocardiogram (ECG) signals acquired from one or more locations on or in a patient. The quantum wave tokenizer system comprises: at least one computing device comprising at least one non-transitory computer readable medium configured to store instructions executable by at least one processor to generate the set of quantum wave tokens, the at least one computing device being configured to: (a) receive the ECG signals; (b) vectorize the ECG signals into one or more vectorized data sets; (c) fit one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal; (d) generate the set of quantum wave tokens corresponding to each of the fitted wave function data sets, each quantum wave token comprising at least one of: an amplitude value, a phase value, an angle value, a width value, and a location value; and (e) provide as an output therefrom at least some of the quantum wave tokens to one or more machine learning models for at least one of: filtering, processing, transforming, analyzing, or diagnosing at least some of the ECG signals.

Yet another aspect of the present disclosure involves a method. The method includes: generating receiving a plurality of electrocardiogram (ECG) signals from a patient; vectorizing the ECG signals into one or more vectorized data sets; fitting one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal; generating a set of quantum wave tokens corresponding to each of the fitted wave function data sets, each quantum wave token comprising at least one of: an amplitude value, a phase value, an angle value, a width value, and a location value; and providing at least some of the quantum wave tokens to one or more machine learning models for at least one of: filtering, processing, transforming, analyzing, or diagnosing at least some of the ECG signals.

One more aspect of the present disclosure involves a method. The method includes: receiving a plurality of electrocardiogram (ECG) signals from a patient; vectorizing the ECG signals into one or more vectorized data sets; fitting one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal; generating a set of quantum wave tokens corresponding to each of the fitted wave function data sets, each quantum wave token comprising at least one of: an amplitude value, a phase value, an angle value, a width value, and a location value; sending the quantum wave tokens to one or more large language models (LLMs); and generating, via the one or more LLMs, a cardiac diagnosis.

The foregoing disclosure is not intended to limit the present disclosure to the precise forms or particular fields of use disclosed. As such, it is contemplated that various alternate embodiments and/or modifications to the present disclosure, whether explicitly described or implied herein, are possible in light of the disclosure. Having thus described embodiments of the present disclosure, persons of ordinary skill in the art will recognize that changes may be made in form and detail without departing from the scope of the present disclosure.

## Claims

1. A quantum wave tokenizer system configured to generate a set of quantum wave tokens corresponding to a plurality of electrocardiogram (ECG) signals acquired from one or more locations on or in a patient, the quantum wave tokenizer system comprising:
at least one computing device comprising at least one non-transitory computer readable medium configured to store instructions executable by at least one processor to generate the set of quantum wave tokens, the at least one computing device being configured to:
(a) receive the ECG signals;
(b) vectorize the ECG signals into one or more vectorized data sets;
(c) fit one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal;
(d) generate the set of quantum wave tokens corresponding to each of the fitted wave function data sets, each quantum wave token comprising at least one of: an amplitude value, a phase value, an angle value, a width value, and a location value; and
(e) provide as an output therefrom at least some of the quantum wave tokens to one or more machine learning models for at least one of: filtering, processing, transforming, analyzing, or diagnosing at least some of the ECG signals.

2. The quantum wave tokenizer system of claim 1, wherein the parametric wave functions comprise one or more of: Gaussian-based parametric wave functions, Slater-Jastrow wave functions, neural network quantum state (NNQS) functions, or functions for minimizing energy expectation values.

3. The quantum wave tokenizer system of claim 1, wherein each ECG signal is segmented into RR intervals prior to being vectorized.

4. The quantum wave tokenizer system of claim 3, wherein each segment of the segmented ECG signal is vectorized to obtain a two-dimensional angle-magnitude representation thereof by fitting an inverse solution for a patient-specific conversion matrix.

5. The quantum wave tokenizer system of claim 3, wherein the location parameter represents:
a temporal or spatial extent of a portion of the ECG signals; or
a temporal offset within one of the RR intervals.

6. The quantum wave tokenizer system of claim 1, the at least one parameter represents one or more of: T-wave alternans, T-wave variability, Q-wave segment duration, ST-segment elevation, prolonged QT-interval, or QRS-segment duration.

7. The quantum wave tokenizer system of claim 1, wherein the ECG signals are vectorized at least in by applying a transformer-based large language model (LLM) to a natural-language text to generate semantic numerical representations.

8. The quantum wave tokenizer system of claim 7, wherein the parametric wave functions are fitted based on semantic attributes extracted from the natural-language text by the transformer-based LLM.

9. The quantum wave tokenizer system of claim 1, wherein the quantum wave tokens represent semantic meaning components of a medical narrative text, clinical narrative descriptions, medical opinions, or psychological evaluations.

10. A method, comprising:
receiving a plurality of electrocardiogram (ECG) signals from a patient;
vectorizing the ECG signals into one or more vectorized data sets;
fitting one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal;
generating a set of quantum wave tokens corresponding to each of the fitted wave function data sets, each quantum wave token comprising at least one of: an amplitude value, a phase value, an angle value, a width value, and a location value; and
providing at least some of the quantum wave tokens to one or more machine learning models for at least one of: filtering, processing, transforming, analyzing, or diagnosing at least some of the ECG signals.

11. The method of claim 10, wherein the parametric wave functions comprise one or more of: Gaussian-based parametric wave functions, Slater-Jastrow wave functions, neural network quantum state (NNQS) functions, or functions for minimizing energy expectation values.

12. The method of claim 10, further comprising segmenting each ECG signal into RR intervals before the vectorizing.

13. The method of claim 12, further comprising: generating a two-dimensional angle-magnitude representation of a vectorized segment of the ECG signal by fitting an inverse solution for a patient-specific conversion matrix.

14. The method of claim 12, wherein the location parameter represents:
a temporal or spatial extent of a portion of the ECG signals; or
a temporal offset within one of the RR intervals.

15. The method of claim 10, the at least one parameter represents one or more of: T-wave alternans, T-wave variability, Q-wave segment duration, ST-segment elevation, prolonged QT-interval, or QRS-segment duration.

16. The method of claim 10, wherein the vectorizing is performed at least in part by applying a transformer-based large language model (LLM) to a natural-language text to generate semantic numerical representations.

17. The method of claim 16, wherein the parametric wave functions are fitted based on semantic attributes extracted from the natural-language text by the transformer-based LLM.

18. The method of claim 10, wherein the quantum wave tokens represent semantic meaning components of a medical narrative text, clinical narrative descriptions, medical opinions, or psychological evaluations.

19. A method, comprising:
receiving a plurality of electrocardiogram (ECG) signals from a patient;
vectorizing the ECG signals into one or more vectorized data sets;
fitting one or more parametric wave functions to each of the one or more vectorized data sets, thereby generating one or more corresponding fitted wave function data sets, wherein each fitted wave function data set includes at least one parameter that includes one or more of: an amplitude parameter, a phase parameter, an angle parameter, a width parameter, or a location parameter of a corresponding ECG signal;
generating a set of quantum wave tokens corresponding to each of the fitted wave function data sets, each quantum wave token comprising at least one of: an amplitude value, a phase value, an angle value, a width value, and a location value;
sending the quantum wave tokens to one or more large language models (LLMs); and
generating, via the one or more LLMs, a cardiac diagnosis.

20. The method of claim 19, wherein:
the one or more LLMs include a first LLM and a second LLM interacting with the first LLM in a feedback loop;
the first LLM generates an initial cardiac diagnosis based on the quantum wave tokens; and
the second LLM generates patches for the initial cardiac diagnosis through the feedback loop.
